# EUROPEAN PATENT SPECIFICATION

(11) **EP 4 274 565 B1**
(45) Date of publication and mention of the grant of the patent: **22.04.2026**
(21) Application number: 22737297.6
(22) Date of filing: 11.01.2022
(51) Int. Cl.: A61K 31/352, A61P 11/00, A61P 11/06, A61P 29/00, A61K 36/48, C12Q 1/6883, A61P 31/12

(54) **SOY ISOFLAVONES FOR USE IN ASTHMA PREVENTION IN A PATIENT HAVING A PLASMINOGEN ACTIVATOR INHIBITOR 1 (PAI-1) RISK GENOTYPE**
SOJAISOFLAVONE ZUR VERWENDUNG BEI ASTHMAVORBEUGUNG BEI EINEM PATIENTEN MIT EINEM PLASMINOGENAKTIVATORINHIBITOR 1 (PAI-1)-RISIKOGENOTYP
ISOFLAVONES DE SOJA POUR UNE UTILISATION DANS LA PRÉVENTION DE L'ASTHME CHEZ UN PATIENT AYANT UN GÉNOTYPE DE RISQUE D'INHIBITEUR 1 D'ACTIVATEUR DU PLASMINOGÈNE (PAI-1)

(30) Priority: 11.01.2021 US 202163136160 P
(43) Date of publication of application: 15.11.2023
(73) Proprietor: University of South Florida, Tampa, FL 33612 (US); Ann and Robert H. Lurie Children's Hospital of Chicago, Chicago, IL 60611-2605 (US)
(72) Inventor: CHO, Seong H., Tampa, Florida 33647 (US); KUMAR, Rajesh, Chicago, Illinois 60618 (US)
(74) Representative: Cabinet Chaillot
(86) International application number: PCT/US2022/011998
(87) International publication number: WO 2022/150770

(56) References cited:
- US-A1- 2004 234 631
- US-A1- 2009 186 038
- US-A1- 2009 186 038
- CHO SEONG H ET AL: "Soy isoflavones reduce asthma exacerbation in asthmatic patients with high PAI-1-producing genotypes", JOURNAL OF ALLERGY AND CLINICAL IMMUNOLOGY, vol. 144, no. 1, July 2019 (2019-07-01), pages 109, XP085726285, ISSN: 0091-6749, DOI: 10.1016/J.JACI.2019.01.020
- DR SEONG HO CHO: "Soy Isoflavones Improve Poor Asthma Control in Asthmatics with High PAI-1 Producing Genotypes", J. ALLERGY CLIN. IMMUNOL., 1 February 2018 (2018-02-01), pages AB400 - AB400, XP055909136
- CHO SEONG H.; JO ARA; CASALE THOMAS; JEONG SU J.; HONG SEUNG-JAE; CHO JOONG K.; HOLBROOK JANET T.; KUMAR RAJESH; SMITH LEWIS J.: "Soy isoflavones reduce asthma exacerbation in asthmatic patients with high PAI-1–producing genotypes", JOURNAL OF ALLERGY AND CLINICAL IMMUNOLOGY, ELSEVIER, AMSTERDAM, NL, vol. 144, no. 1, 1 January 1900 (1900-01-01), AMSTERDAM, NL , pages 109, XP085726285, ISSN: 0091-6749, DOI: 10.1016/j.jaci.2019.01.020
- HUANG X., YANG M, WANG Y, ZHANG X, YANG H,: "Association between the Promoter -675 4G/5G Polymorphism of the Plasminogen Activator Inhibitor-1 Gene and Asthma: An Update of Meta-Analysis", AUSTIN IMMUNOLOGY, 10 November 2016 (2016-11-10), XP055956142, [retrieved on 20220830]

## Description

### CROSS REFERENCE TO RELATED APPLICATIONS

This application is a nonprovisional of and claims priority to U.S. Provisional Patent Application Serial No. 63/136,160, entitled "Soy Isoflavones for Asthma Prevention", filed January 11,

### FIELD OF INVENTION

This invention relates to prevention of asthma. Specifically, the invention provides a method of preventing asthma by the administration of soy isoflavones, as defined in the enclosed claims. Any references in the description to methods of treatment refer to the compounds, pharmaceutical compositions and medicaments of the invention for use in a method of treatment of the human body by therapy.

### BACKGROUND OF THE INVENTION

Asthma is a chronic disease affecting more than 24 million persons in the United States. Airway remodeling, one of the main pathologic hallmarks of uncontrolled asthma and fatalities, is characterized by subepithelial fibrosis, goblet cell hyperplasia, and increased airway smooth muscle cell mass. (Durrani SR, Viswanathan RK, Busse WW. What effect does asthma treatment have on airway remodeling? Current perspectives. J Allergy Clin Immunol 2011;128:439-48) Airway remodeling is associated with a decrease in lung function and excessive airway narrowing. (James AL, Maxwell PS, Pearce-Pinto G, Elliot JG, Carroll NG. The relationship of reticular basement membrane thickness to airway wall remodeling in asthma. Am J Respir Crit Care Med 2002;166:1590-5; McParland BE, Macklem PT, Pare PD. Airway wall remodeling: friend or foe? J Appl Physiol 2003;95:426-34; Pare PD, McParland BE, Seow CY. Structural basis for exaggerated airway narrowing. Can J Physiol Pharmacol 2007;85:653-8; Royce SG, Tan L, Koek AA, Teng ML. Effect of extracellular matrix composition on airway epithelial cell and fibroblast structure: implications for airway remodeling in asthma. Ann Allergy Asthma Immunol 2009;102:238-46) Patients with fatal asthma exhibit more severe airway remodeling than those with nonfatal asthma, and airway remodeling is attributed to treatment failure with conventional medications, such as inhaled corticosteroids, and poor asthma control. (James AL, Maxwell PS, Pearce-Pinto G, Elliot JG, Carroll NG. The relationship of reticular basement membrane thickness to airway wall remodeling in asthma. Am J Respir Crit Care Med 2002;166:1590-5; Chetta A, Foresi A, Del Donno M, Bertorelli G, Pesci A, Olivieri D. Airways remodeling is a distinctive feature of asthma and is related to severity of disease. Chest 1997;111:852-7). Therefore therapeutic approaches targeting airway remodeling would be vital in reducing asthma morbidity and mortality.

Plasminogen activator inhibitor 1 (PAI-1) is associated with asthma severity because of its role in airway remodeling. (Cho SH, Ryu CH, Oh CK. Plasminogen activator inhibitor-1 in the pathogenesis of asthma. Exp Biol Med (Maywood) 2004;229:138-46). Plasminogen is converted to plasmin by tissue-type plasminogen activator or urokinase-type plasminogen activator. Both plasminogen activators are involved in dissolution of fibrin and degradation of extracellular matrix (ECM) components. (Rijken DC, Sakharov DV. Basic principles in thrombolysis: regulatory role of plasminogen. Thromb Res 2001;103:S41-9). PAI-1 is the primary inhibitor for both tissue-type plasminogen activator and urokinase-type plasminogen activator. Previously, the inventors demonstrated that deletion of PAI-1 prevents ECM deposition in a murine model of chronic asthma and that blocking PAI-1 with a specific inhibitor or small interfering RNA reduces airway inflammation, tissue remodeling, and airway hyperresponsiveness. (Oh CK, Ariue A, Alban RF, Shaw B, Cho SH. PAI-1 promotes extracellular matrix deposition in the airways of a murine asthma model. Biochem Biophys Res Commun 2002;294:1055-60; Miyamoto S, Hattori N, Senoo T, Onari Y, Iwamoto H, Kanehara M, et al. Intra-airway administration of small interfering RNA targeting plasminogen activator inhibitor-1 attenuates allergic asthma in mice. Am J Physiol Lung Cell Mol Physiol 2011;301:L908-16; Lee SH, Eren M, Vaughan DE, Schleimer RP, Cho SH. A plasminogen activator inhibitor-1 inhibitor reduces airway remodeling in a murine model of chronic asthma. Am J Respir Cell Mol Biol 2012;46:842-6) This suggests that PAI-1 plays a key role in deposition of ECM in the airways and might be implicated in asthma severity and outcomes. Therefore PAI-1 can be an important target for therapeutic solutions to asthma.

Soy has been studied as a potential therapeutic option for asthma. Genistein and daidzein, 2 major soy isoflavones, have been investigated for their role in asthma. Previous studies in asthmatic patients have demonstrated that consumption of soy isoflavones results in better lung function. (Smith LJ, Holbrook JT, Wise R, Blumenthal M, Dozor AJ, Mastronarde J, et al. Dietary intake of soy genistein is associated with lung function in patients with asthma. J Asthma 2004;41:833-43; Bime C, Wei CY, Holbrook J, Smith LJ, Wise RA. Association of dietary soy genistein intake with lung function and asthma control: a post-hoc analysis of patients enrolled in a prospective multicentre clinical trial. Prim Care Respir J 2012;21:398-404).

However, a recent multicenter, placebo-controlled, randomized clinical trial study examining use of a soy isoflavone supplement compared with placebo did not result in improved lung function or clinical outcomes in the entire population. (Smith LJ, Kalhan R, Wise RA, Sugar EA, Lima JJ, Irvin CG, et al. Effect of a soy isoflavone supplement on lung function and clinical outcomes in patients with poorly controlled asthma: a randomized clinical trial. JAMA 2015;313: 2033-43).

One of the reasons for the negative results in the soy isoflavone trial might be intrinsic individual differences in medication responses because of genetic variation. *In vitro* and animal studies have reported an association between soy intake and changes in PAI-1 levels, with a soy protein diet in rats reducing expression of PAI-1 in adipose tissue. (Nagasawa A, Fukui K, Kojima M, Kishida K, Maeda N, Nagaretani H, et al. Divergent effects of soy protein diet on the expression of adipocytokines. Biochem Biophys Res Commun 2003;311:909-14; van Hinsbergh VW, Vermeer M, Koolwijk P, Grimbergen J, Kooistra T. Genistein reduces tumor necrosis factor alpha-induced plasminogen activator inhibitor-1transcription but not urokinase expression in human endothelial cells. Blood 1994;84:2984-91). These studies suggest that soy isoflavones might be more effective in asthmatic patients with ahigh PAI-1 level.

The PAI-1 gene resides on chromosome 7, and several polymorphisms in this gene have been found. In particular, a single guanosine insertion/deletion variation at position 2675 in the promoter region of the PAI-1 gene (rs1799768, 4G or 5G) has been extensively studied. It has been found that plasma PAI-1 levels are greater in patients with the 4G4G genotype than in those with the 5G5G genotype, with the 4G5G group having intermediate values. (Tsantes AE, Nikolopoulos GK, Bagos PG, Bonovas S, Kopterides P, Vaiopoulos G. The effect of the plasminogen activator inhibitor-1 4G/5G polymorphism on the thrombotic risk. Thromb Res 2008;122:736-42).

The inventors first reported an association between the 4G5G polymorphism and asthma development, and the 4G4G genotype was found to be associated with decreased FEV₁ in house dust mite-sensitive allergic asthmatic patients. (Cho SH, Hall IP, Wheatley A, Dewar J, Abraha D, Del Mundo J, et al. Possible role of the 4G/5G polymorphism of the plasminogen activator inhibitor 1 gene in the development of asthma. J Allergy Clin Immunol 2001; 108:212-4; Pampuch A, Kowal K, Bodzenta-Lukaszyk A, Di Castelnuovo A, Chyczewski L, Donati MB, et al. The -675 4G/5G plasminogen activator inhibitor-1 promoter polymorphism in house dust mite-sensitive allergic asthma patients. Allergy 2006;61:234-8). The recent studies with a large patient cohort showed a negative association between a high PAI-1-producing genotype and lung function/asthma severity. (Cho SH, Min JY, Kim DY, Oh SS, Torgerson DR, Pino-Yanes M, et al. Association of a PAI-1 gene polymorphism and early life infections with asthma risk, exacerbations, and reduced lung function. PLoS One 2016;11: e0157848). The inventors previously showed differences in the effects of soy isoflavones on asthma exacerbation based on PAI-1 polymorphisms and resultant differential PAI-1 levels using data and DNA/blood samples from the published Study of Soy Isoflavones in Asthma (SOYA) trial. (Smith LJ, Kalhan R, Wise RA, Sugar EA, Lima JJ, Irvin CG, et al. Effect of a soy isoflavone supplement on lung function and clinical outcomes in patients with poorly controlled asthma: a randomized clinical trial. JAMA 2015;313: 2033-43).

### Pediatric Asthma

Pediatric asthma results in significant cost to families and society ($5.92 billion annually) due to increased risk for emergency department visits, hospitalization, and work/school absenteeism. There is no proven asthma prevention strategy, possibly due to the fact that there are multiple asthma endotypes. A precision medicine approach may be necessary for prevention. The inventors found that a frequent gain of function promoter polymorphism for the PAI-1 gene (~60% of the population are homozygous or heterozygous) is associated with elevated circulating PAI-1 levels. In studies of established asthma, the gain of function genotype is associated with higher rates of asthma exacerbations, worse asthma control, and lower lung function. Furthermore, if children homo or heterozygous for the risk allele had a respiratory viral illness requiring an MD visit before age 2, they had a 12 (any virus) to 18-fold (RSV) increased risk of asthma.

The mechanisms responsible for this increase in risk of developing asthma are not known, but may be related to shifting the airway to a Th2 endotype at a critical point in time. The inventors demonstrated that PAI-1 levels increase in the airway of asthmatic subjects in response to viral illness. While PAI-1 has been shown to be associated with airway remodeling, the inventors also showed that a PAI-1 specific inhibitor reduced airway eosinophilia and that mast cell derived PAI-1 is associated with airway thymic stromal lymphopoietin (TSLP) production. TSLP production after viral insult /TLR3 stimulation is well established. TSLP promotes Th2 airway endotypes by enhancing IgE isotype switching and the production of chemokines (eotaxin-2/CCL24 and TARC/CCL17), which recruit eosinophils and Th2 cells to the airway. It is not known if these effects extend beyond the viral episode, or if there is a critical window in which they are particularly influential.

The inventors have also shown increased total serum IgE levels in subjects with the high PAI-1 producing genotype, suggesting a relationship between PAI-1 and IgE. This IgE elevation associated with PAI-1 may increase FcεRI levels on pDCs which attenuate type I interferon antiviral responses. If PAI-1 is decreased it may both affect airway endotype in response to virus and decrease PAI-1/IgE feedback, providing two ways to potentially modulate progression to asthma. PAI-1 associated airway inflammation may be interrupted by soy isoflavones such as genistein. The inventors have shown that soy isoflavones reduce TGF-β1-induced PAI-1 gene expression and protein production from airway epithelial cells. Furthermore, the inventors found that subjects with asthma and the PAI-1 risk genotype who received soy isoflavones had a 70% reduction in severe asthma exacerbations. It has not previously been determined whether dietary administration of soy in young infants decreases Th2 type inflammation in the airway after viral illness, reduces IgE production and decreases the development of asthma.

The inventors use a precision medicine approach directed to a genotype that is relatively common in the population, and as such, impacts a large number of children at risk of developing asthma. While soy isoflavones such as soy genistein are bioactive as selective estrogen receptor modulators, these levels of ingestion have been found to have a good safety profile. (Ogawa M, Kitano T, Kawata N, et al. Daidzein down-regulates ubiquitin-specific protease 19 expression through estrogen receptor beta and increases skeletal muscle mass in young female mice. The Journal of nutritional biochemistry 2017;49:63-70). Using an inexpensive, safe food derived therapy is a departure from current medication based attempts to prevent asthma.

While omalizumab is currently being studied as an immunomodulatory therapy to prevent asthma, it remains to be seen if this approach is effective, and if so whether it is cost effective. Omalizumab is a costly biological agent (annual cost of $10,000-16,000/year), administered by injection, and must be administered in a medical professional's office. (Lieberman PL, Jones I, Rajwanshi R, Rosen K, Umetsu DT. Anaphylaxis associated with omalizumab administration: Risk factors and patient characteristics. The Journal of allergy and clinical immunology 2017;140:1734-6 e4). Another approach for preventing asthma is disclosed in US 2009/186038 A1. In contrast, soy isoflavones are safe, inexpensive with a cost estimated at 8 cents/day, and is being targeted as a precision medicine intervention for 60% of the population with the risk genotype
Given the lack of effective and affordable preventative methods for asthma, what is needed is a composition that is efficacious as a preventative for asthma.

### SUMMARY OF INVENTION

In an embodiment, a method of preventing asthma development in a patient having a plasminogen activator inhibitor 1 (PAI-1) risk genotype is presented comprising: determining or having determined a PAI-1 genotype of the patient and administering a therapeutically effective amount of at least one soy isoflavone to the patient if the PAI-1 risk genotype is 4G4G or 4G5G. The administration of the therapeutically effective amount of the at least one soy isoflavone to the patient prevents the asthma development. The patient may be an infant.

The at least one soy isoflavone may be selected from the group consisting of genistein, daidzein, and glycitein. The at least one soy isoflavone may administered to the patient orally. The at least one soy isoflavone may also be administered daily, particularly throughout a viral season.

In another embodiment, a method of preventing asthma development in a pediatric patient having a plasminogen activator inhibitor 1 (PAI-1) risk genotype is presented comprising: determining or having determined a PAI-1 risk genotype of the pediatric patient and administering a therapeutically effective amount of at least one soy isoflavone to the patient if the PAI-1 risk genotype of the patient is 4G4G or 4G5G. The administration of the therapeutically effective amount of the at least one soy isoflavone to the pediatric patient prevents the asthma development.

The pediatric patient may be at high risk of the asthma development due to uni- or biparental asthma or atopic dermatitis. The at least one soy isoflavone may be selected from the group consisting of genistein, daidzein, and glycitein. The at least one soy isoflavone may administered to the patient orally. The at least one soy isoflavone may also be administered daily, particularly throughout a viral season.

In a further embodiment, a method of determining responsiveness to soy isoflavone administration for prevention of asthma development in a patient in need thereof is presented comprising: determining or having determined a plasminogen activator inhibitor 1 (PAI-1) risk genotype of the patient and administering a therapeutically effective amount of at least one soy isoflavone to the patient prior to the development of the asthma if the PAI-1 risk genotype is 4G4G or 4G5G. The PAI-1 risk genotype of 4G4G or 4G5G is responsive to the prevention of the asthma in the patient.

The at least one soy isoflavone may be selected from the group consisting of genistein, daidzein, and glycitein and administered to the patient orally.

### BRIEF DESCRIPTION OF THE DRAWINGS

For a fuller understanding of the invention, reference should be made to the following detailed description, taken in connection with the accompanying drawings, in which:
**Fig. 1A-B** are a series of images depicting TGF-β1-induced production of PAI-1 gene and protein in NHBE cells. NHBE cells were cultured and stimulated with recombinant human TGF-β1 (2 ng/mL) with and without genistein pretreatment (1 or 5 µmol/L) for 1 hour. Cell pellets were used for real-time PCR and cell-culture supernatants for PAI-1 protein ELISA.
**Fig. 2** is an image depicting soy isoflavones reduce asthma exacerbation in high PAI-1 producing genotype.
**Fig. 3** is a graph depicting the odds of asthma by RSV and genotype.
**Fig. 4A-B** are a series of graphs depicting PAI-1 augments IL-4/TLR3 induced TSLP production. **(A)** graph of relative expression of TSLP mRNA; and **(B)** graph of TSLP amount.
**Fig. 5** is a graph depicting genistein significantly reduces PAI-1 production from human bronchial EC and mast cells.
**Fig. 6** is an image depicting nasal and bronchial transcriptomes.
**Fig. 7** is an image depicting network analysis of T2 high gene expression.
**Fig. 8** is an image depicting the log total in GALA2 cohort by PAI-1 promotor genotype.
**Fig. 9** is an image depicting the experimental plan for Example 3.
**Fig. 10A-C** are a series of graphs depicting the total respiratory resistance (Rrs) used to assess airway hyperresponsiveness (AHR). (A) graph illustrating the Rrs for each group ((PBS+Veh; PBS+Bio300; OVA+Veh; OVA+Bio300) as a percentage of baseline with increasing amounts of methacholine; **(B)** graph illustrating the Rrs for each group as a percentage of baseline with administration of 25 mg/ml methacholine; and **(C)** graph illustrating the Rrs for each group as a percentage of baseline with administration of 50 mg/ml methacholine.
**Fig. 11A-E** are a series of graphs depicting the cell count in bronchoalveolar lavage fluid analysis (BALF). **(A)** total cell count in BALF; **(B)** lymphocyte cell count in BALF; **(C)** eosinophil cell count in BALF; **(D)** neutrophil cell count in BALF; and **(E)** eosinophils/lymphocytes ratio in BALF.

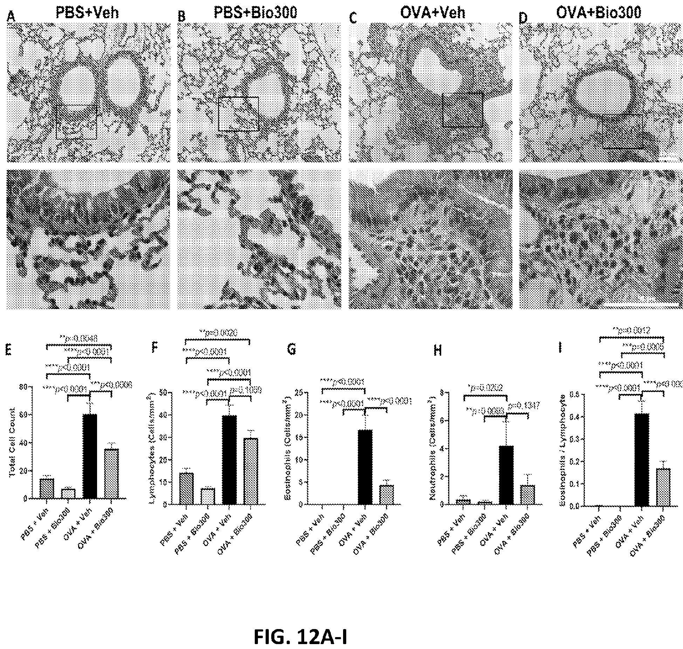

**Fig. 12A-I** are a series of images illustrating cell counts in tissue for the various groups. **(A)** tissue staining of the PBS+Veh group; **(B)** tissue staining for the PBS+Bio300 group; **(C)** tissue staining for the OVA+Veh group; **(D)** tissue staining for the OVA+Bio300 group; **(E)** graph of the total cell count for each group (PBS+Veh; PBS+Bio300; OVA+Veh; OVA+Bio300); **(F)** graph of the number of lymphocytes for each group; **(G)** graph of the number of eosinophils for each group; **(H)** graph of the number of neutrophils for each group; and **(I)** graph of the ratio of eosinophils/lymphocytes for each group.
**Fig. 13A-B** area series of images depicting goblet cells via PAS staining. **(A)** PAS staining for each group (PBS+Veh; PBS+Bio300; OVA+Veh;
OVA+Bio300); and **(B)** graph depicting the percentage of PAS cell count.
**Fig. 14** is an image depicting the soy isoflavone and PAI-1 immune deviation study.

### DETAILED DESCRIPTION OF THE PREFERRED EMBODIMENT

In the following detailed description of the preferred embodiments, reference is made to the accompanying drawings, which form a part hereof, and within which are shown by way of illustration specific embodiments by which the invention may be practiced. It is to be understood that other embodiments may be utilized, and structural changes may be made without departing from the scope of the invention.

### Abbreviations

ACT - Asthma Control Test
ASUI - Asthma Symptoms Utility index
ECM - Extracellular Matrix
NHBE - Normal Human Bronchial Epithelial
PAI-1 - Plasminogen activator inhibitor 1
SNP - Single Nucleotide Polymorphism
SOYA - Study of Soy Isoflavones in Asthma

### Definitions

Unless otherwise defined, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are described herein. All publications mentioned herein are incorporated herein by reference in their entirety to disclose and describe the methods and/or materials in connection with which the publications are cited. It is understood that the present disclosure supercedes any disclosure of an incorporated publication to the extent there is a contradiction.

As used herein, the singular forms "a," "an" and "the" include plural referents unless the context clearly dictates otherwise.

As used in this specification and the appended claims, the term "or" is generally employed in its sense including "and/or" unless the context clearly dictates otherwise.

All numerical designations, such as pH, temperature, time, concentration, and molecular weight, including ranges, are approximations which are varied up or down by increments of 1.0, 0.1, 0.01 or 0.001 as appropriate. It is to be understood, even if it is not always explicitly stated that all numerical designations are preceded by the term "about". It is also to be understood, even if it is not always explicitly stated, that the reagents described herein are merely exemplary and that equivalents of such are known in the art and can be substituted for the reagents explicitly stated herein.

As used herein, the term "comprising" is intended to mean that the products, compositions, and methods include the referenced components or steps, but not excluding others. "Consisting essentially of" when used to define products, compositions, and methods, shall mean excluding other components or steps of any essential significance. Thus, a composition consisting essentially of the recited components would not exclude trace contaminants and pharmaceutically acceptable carriers. "Consisting of" shall mean excluding more than trace elements of other components or steps.

As used herein, "about" means approximately or nearly and in the context of a numerical value or range set forth means ± 15% of the numerical.

As used herein "patient" is used to describe an animal, preferably a human, to whom treatment is administered, including prophylactic treatment with the compositions of the present invention. "Patient" and "subject" are used interchangeably herein.

As used herein "animal" means a multicellular, eukaryotic organism classified in the kingdom Animalia or Metazoa. The term includes, but is not limited to, mammals. Non-limiting examples include rodents, mammals, aquatic mammals, domestic animals such as dogs and cats, farm animals such as sheep, pigs, cows and horses, and humans. Wherein the terms "animal" or the plural "animals" are used, it is contemplated that it also applies to any animals.

"Administering" or "administration" as used herein refers to the process by which the compositions of the present invention are delivered to the patient. The compositions may be administered in various ways, including but not limited to, orally, nasally, rectally, and parenterally.

A "therapeutically effective amount" as used herein is defined as concentrations or amounts of components which are sufficient to effect beneficial or desired clinical results, including, but not limited to, any one or more of treating symptoms of asthma and preventing asthma. Compositions of the present invention can be used to effect a favorable change in the condition whether that change is an improvement, such as stopping, reversing, or reducing asthma, or a complete elimination of symptoms or prevention of symptoms due to asthma. In accordance with the present invention, a suitable single dose size is a dose that is capable of preventing or alleviating (reducing or eliminating) a symptom in a patient when administered one or more times over a suitable time period. One of skill in the art can readily determine appropriate single dose sizes for systemic administration based on the size of the animal and the route of administration. In some embodiments, the effective amount may range from about 0.01 mg/kg to about 10 mg/kg of at least one soy isoflavone per day, including all amounts in between. In some embodiments, the amount of soy isoflavone per day is between about 1.3 - 6.2 mg/kg/day. The dose may be adjusted according to response.

The amount of the compound in the drug composition will depend on absorption, distribution, metabolism, and excretion rates of the drug as well as other factors known to those of skill in the art. Dosage values may also vary with the severity of the condition to be alleviated. The compounds may be administered once, or may be divided and administered over intervals of time. It is to be understood that administration may be adjusted according to individual need and professional judgment of a person administrating or supervising the administration of the compounds used in the present invention.

The dose of the compounds administered to a subject may vary with the particular composition, the method of administration, and the particular disorder being treated. The dose should be sufficient to affect a desirable response, such as a therapeutic or prophylactic response against a particular disorder or condition. It is contemplated that one of ordinary skill in the art can determine and administer the appropriate dosage of compounds disclosed in the current invention according to the foregoing considerations.

Dosing frequency for the composition includes, but is not limited to, at least about once every three weeks, once every two weeks, once a week, twice a week, three times a week, four times a week, five times a week, six times a week, or daily. In some embodiments, the interval between each administration is less than about a week, such as less than about any of 6, 5, 4, 3, 2, or 1 day. In some embodiments, the interval between each administration is constant. For example, the administration can be carried out daily, every two days, every three days, every four days, every five days, or weekly. In some embodiments, the administration can be carried out twice daily, three times daily, or more frequent. Administration can also be continuous and adjusted to maintaining a level of the compound within any desired and specified range.

The administration of the composition can be extended over an extended period of time, such as from about a month or shorter up to about three years or longer. For example, the dosing regimen can be extended over a period of any of about 2, 3, 4, 5, 6, 7, 8, 9, 10, 11, 12, 18, 24, 30, and 36 months. In some embodiments, there is no break in the dosing schedule. In some embodiments, the interval between each administration is no more than about a week.

The compounds used in the present invention may be administered individually, or in combination with or concurrently with one or more other compounds used against asthma. Additionally, compounds used in the present invention may be administered in combination with or concurrently with other therapeutics or preventatives for asthma or other respiratory viruses.

"Prevention" or "preventing" as used herein refers to any of: halting the effects of asthma, reducing the effects of asthma, reducing the incidence of asthma, reducing the development of asthma, delaying the onset of symptoms of asthma, increasing the time to onset of symptoms of asthma, and reducing the risk of development of asthma.

"Treatment" or "treating" as used herein refers to any of the alleviation, amelioration, elimination and/or stabilization of a symptom, as well as delay in progression of a symptom of a particular disorder. For example, "treatment" of asthma may include any one or more of the following: amelioration and/or elimination of one or more symptoms associated with asthma, reduction of one or more symptoms of asthma, stabilization of symptoms of asthma, and delay in progression of one or more symptoms of asthma.

The terms "risk or susceptibility" as used herein refers to the determination as to whether a subject would or would not respond to a particular therapy or would or would not develop a particular disease or symptom.

The pharmaceutical compositions of the subject invention can be formulated according to known methods for preparing pharmaceutically useful compositions. Furthermore, as used herein, the phrase "pharmaceutically acceptable carrier" means any of the standard pharmaceutically acceptable carriers. The pharmaceutically acceptable carrier can include diluents, adjuvants, and vehicles, as well as implant carriers, and inert, non-toxic solid or liquid fillers, diluents, or encapsulating material that does not react with the active ingredients of the invention. Examples include, but are not limited to, phosphate buffered saline, physiological saline, water, and emulsions, such as oil/water emulsions. The carrier can be a solvent or dispersing medium containing, for example, ethanol, polyol (for example, glycerol, propylene glycol, liquid polyethylene glycol, and the like), suitable mixtures thereof, and vegetable oils. Formulations are described in a number of sources that are well known and readily available to those skilled in the art. For example, Remington's Pharmaceutical Sciences (Martin EW [1995] Easton Pennsylvania, Mack Publishing Company, 19th ed.) describes formulations which can be used in connection with the subject invention. In some embodiments, particularly those for use in oral administration to infants, the pharmaceutically acceptable carrier can be infant formula in either powder or liquid forms.

For ease of administration, the subject compounds may be formulated into various pharmaceutical forms. These pharmaceutical compositions are desirably in unitary dosage form suitable, preferably, for administration nasally, orally, rectally, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules often represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution.

"Composition" as used herein refers to a product comprising the specified ingredients in the specified amounts, as well as any product which results, directly or indirectly, from combination of the specified ingredients in the specified amounts. In some embodiments, the composition includes both the therapeutic agent as well as one or more pharmaceutically acceptable carriers.

"Soy isoflavones" as used herein refers to heterocyclic phenols having structural similarity to estradiol-17β and selective estrogen receptor modulators. Soy isoflavones are derived from soybeans. Exemplary soy isoflavones useful in the instant invention include, but are not limited to, genistein, daidzein, and glycitein

"Viral season" as used herein refers to the time of year in which virus infections are the most prevalent, generally beginning in the fall and lasting throughout winter.

The 4G4G genotype of plasminogen activator inhibitor 1 (PAI-1) is associated with increased plasma PAI-1 levels and poor asthma control. Previous studies suggest that soy isoflavones can reduce PAI-1 levels. A PAI-1 functional polymorphism (rs1799768, 4G5G) was characterized in subjects with poorly controlled asthma enrolled in a randomized clinical trial of soy isoflavones (n=265) Genotype-specific treatment responses on asthma outcomes were compared between soy isoflavones and placebo. Normal human bronchial epithelial cells were cultured with or without TGF-β1, genistein, or both, and PAI-1 levels were measured. The 4G4G/4G5G genotype was associated with a greater risk for allergy-related worsened asthma symptoms and eczema at baseline compared with the 5G5G genotype. There was a significant interaction between the genotype and soy isoflavone intervention on oral corticosteroid use for asthma exacerbation (*P* = .005). In a subgroup analysis soy isoflavones significantly reduced the use of oral corticosteroids (number of events/person-year) by 4-fold compared with placebo in the 4G4G/4G5G genotype (0.2 vs 0.8; relative risk, 0.28; *P* < .001) but not in the 5G5G genotype. Soy isoflavones reduced plasma PAI-1 levels compared with placebo. Genistein treatment reduced TGF-β1-induced PAI-1 production in normal human bronchial epithelial cells. Soy isoflavone treatment provides a significant benefit in reducing the number of severe asthma exacerbations in asthmatic patients with the high PAI-1-producing genotype. PAI-1 polymorphisms can be used as a genetic biomarker for soy isoflavone-responsive patients with asthma.

In addition to the findings above, the inventors sought to determine 1) whether soy isoflavones can modulate development of Th2 airway endotypes, decrease eosinophilic airway inflammation, and decrease sensitization in high risk infants with the PAI-1 risk genotype, and 2) interrupt the PAI-1-IgE axis, and thereby improve rhinovirus-induced IFN-α response. The inventors believe that if children with the PAI-1 risk genotype (homo or heterozygous) with a severe viral illness are being treated with soy isoflavones during peak viral seasons, they will have less production of PAI-1 in the airway, a decreased risk of developing Th2 airway endotypes, and restoration of the virus-induced IFN-α response.

The inventors have developed a precision medicine approach, which is directed to a genotype that is relatively common, and as such, could impact a large number of children at risk of developing asthma. While soy isoflavones such as soy genistein are bioactive as selective estrogen receptor modulators, these levels of ingestion have been found to have a good safety profile. (Ogawa M, Kitano T, Kawata N, et al. Daidzein down-regulates ubiquitin-specific protease 19 expression through estrogen receptor beta and increases skeletal muscle mass in young female mice. The Journal of nutritional biochemistry 2017;49:63-70). Using an inexpensive, safe food derived therapy is a departure from current medication based attempts to prevent asthma. While omalizumab is currently being studied as an immunomodulatory therapy to prevent asthma, it remains to be seen if this approach is effective, and if so whether it is cost effective. Omalizumab is a costly biological agent (annual cost of $10,000-16,000/year), administered by injection, and must be administered in a medical professional's office. (Lieberman PL, Jones I, Rajwanshi R, Rosen K, Umetsu DT. Anaphylaxis associated with omalizumab administration: Risk factors and patient characteristics. The Journal of allergy and clinical immunology 2017;140:1734-6 e4). In contrast, soy isoflavones are safe, inexpensive with a cost estimated at 8 cents/day, and are being targeted as a precision medicine intervention for 60% of the population with the risk genotype.

The following non-limiting examples illustrate exemplary systems and components thereof in accordance with various embodiments of the disclosure. The examples are merely illustrative and are not intended to limit the disclosure in any way.

### Example 1 - Soy isoflavones reduce asthma exacerbation in asthmatic patients with high PAI-1-producing genotypes

Asthma is a complex and heterogeneous disease, and its severity is influenced by genetic and environmental factors. Diet is one of the environmental factors, and dietary modification or dietary supplements have been considered for their potential clinical benefit in asthma management. (Raviv S, Smith LJ. Diet and asthma. Curr Opin Pulm Med 2010;16:71-6). However, most clinical trials using dietary supplements have not been successful in improving clinically relevant outcomes, including a recent multicenter placebo-controlled, randomized clinical trial with a soy isoflavone supplement. This failure suggests that a potential benefit might be limited to a subgroup of asthmatic patients with specific phenotypic or genotypic characteristics related to the biologic target of soy isoflavones. The inventors demonstrated that soy isoflavone treatment indeed shows a significant benefit in reducing the number of severe asthma exacerbations in asthmatic patients with 4G4G/4G5G PAI-1 genotypes but not in those with the 5G5G genotype. In support of this clinical difference, there were significant decreases in plasma PAI-1 levels by soy isoflavone treatment, suggesting a biologic connection between genistein intake and plasma PAI-1 levels. Furthermore, downregulation of TGF-b1-induced PAI-1 production from normal bronchial epithelial cells by genistein treatment suggests that genistein can reduce PAI-1 production in the airways of asthmatic patients.

Viral infection and allergen exposure are known to be the main triggers of asthma exacerbations. (Jackson DJ, Gangnon RE, Evans MD, Roberg KA, Anderson EL, Pappas TE, et al. Wheezing rhinovirus illnesses in early life predict asthma development in high-risk children. Am J Respir Crit Care Med 2008;178:667-72). Previously, the inventors demonstrated that sputum levels of PAI-1 are significantly increased in asthmatic patients compared with healthy control subjects at baseline, and sputum PAI-1 levels are further increased compared with baseline values during the peak of cold symptoms. (Cho SH, Hong SJ, Chen H, Habib A, Cho D, Lee SH, et al. Plasminogen activator inhibitor-1 in sputum and nasal lavage fluids increases in asthmatic patients during common colds. J Allergy Clin Immunol 2014;133:1465-7). This suggests a role for PAI-1 in virus-induced asthma exacerbations. Another pediatric study also showed increased plasma PAI-1 levels in young children with a history of repeated wheeze. (Lee Chung H, Kim SY, Kim SG. Vascular endothelial growth factor and plasminogen activator inhibitor-1 in children with recurrent early wheeze. J Allergy Clin Immunol 2007;119:1541-2). Among children, PAI-1 levels are even greater in those with more frequent wheezing relapses.

The inventors also showed a harmful interaction between high PAI-1-producing genotypes and lower respiratory tract infections in the development of a more severe phenotype of asthma in the Genes-environments & Admixture in Latino Americans cohort. (Cho SH, Min JY, Kim DY, Oh SS, Torgerson DR, Pino-Yanes M, et al. Association of a PAI-1 gene polymorphism and early life infections with asthma risk, exacerbations, and reduced lung function. PLoS One 2016;11: e0157848). Therefore soy isoflavone treatment can reduce asthma exacerbations, at least in part, by downregulating PAI-1 levels in the airways of patients with asthma.

Here, the 4G4G/4G5G genotype is associated with an increased risk of eczema and allergies associated with worse asthma at baseline. Otherwise, no significant differences in baseline clinical outcomes were observed, including lung function. Previous studies have shown that the high PAI-1-producing4G4G genotype is associated with reduced lung function (FEV₁) and airways hyperresponsiveness in allergic asthmatic patients. (Pampuch A, Kowal K, Bodzenta-Lukaszyk A, Di Castelnuovo A, Chyczewski L, Donati MB, et al. The -675 4G/5G plasminogen activator inhibitor-1 promoter polymorphism in house dust mite-sensitive allergic asthma patients. Allergy 2006;61:234-8). The inventors previously demonstrated that a high PAI-1-producing genotype (rs2227631; AA/AG) is associated with lower FEV₁/forced vital capacity percent predicted and a greater risk for asthma-related hospitalization compared with the wild-type genotype (GG). (Cho SH, Min JY, Kim DY, Oh SS, Torgerson DR, Pino-Yanes M, et al. Association of a PAI-1 gene polymorphism and early life infections with asthma risk, exacerbations, and reduced lung function. PLoS One 2016;11: e0157848). Both studies were conducted in white and His- panic populations. Here, 43% of the study population is black, which is likely reflected in the greater prevalence of the 5G5G genotype (37%) compared with about 25% of the 5G5G genotype in a white population. (Festa A, D'Agostino R Jr, Rich SS, Jenny NS, Tracy RP, Haffner SM. Promoter (4G/5G) plasminogen activator inhibitor-1 genotype and plasminogen activator inhibitor-1 levels in blacks, Hispanics, and non-Hispanic whites: the Insulin Resistance Atherosclerosis Study. Circulation 2003;107:2422-7). Although ethnicity was adjusted for in the analyses, there may be some residual confounding from racial differences.

The inventors found no significant difference in baseline plasma PAI-1 levels between the 4G4G/4G5G and 5G5G genotypes. There was also no significant difference in changes of plasma PAI-1 levels with soy isoflavone treatment between the 2 genotype groups. This may be due to wide individual variations of plasma PAI-1 levels and diurnal variation of plasma PAI-1 levels with various blood collection times and/or because blood PAI-1 levels do not accurately reflect airway levels. Thus, soy isoflavone treatment can reduce PAI-1 production equally in both genotype groups in the blood. Soy isoflavones may cause more reduction in the airways during virus-induced asthma exacerbations in the 4G4G/4G5G genotype groups.

Other than new oral corticosteroid use, there were no significant changes in the other clinical outcomes or biomarkers with soy isoflavone treatment compared with placebo control treatment. There were no significant differences in other asthma exacerbation components, such as rescue medication use or peak flow changes, between the soy isoflavone and placebo treatment groups. Oral corticosteroids have been used in multiple clinical trials and clinical guidelines as a marker of a severe asthma exacerbation. In this *post hoc* analysis, the inventors speculate that PAI-1 production is further increased by viral infection or other asthma triggers in patients who already have high PAI-1 production in the airways by virtue of their genetic mutation. Soy isoflavones can alleviate this tipping point by reducing PAI-1 production in those with high baseline levels. Other clinical outcomes or biomarkers are not directly related or not robust enough in this *post hoc* analysis. There was a trend of increased urgent care (unscheduled contact with health care providers) with soy isoflavone treatment in the 4G4G/4G5G group, but there was no significant interaction between soy isoflavones and the polymorphism in an interaction analysis regarding urgent care visits. Furthermore, the total number of urgent care contacts was 32, and among these 32 contacts, only 4 resulted in taking oral corticosteroids, indicating that most of these unscheduled contacts were not severe asthma exacerbations

### Materials and Methods

### Study Design and Subjects

SOYA was a randomized clinical trial conducted at 19 clinical centers in the United States from May 2010 through August 2012 and previously published in Smith et al. 2015. In the present study the inventors investigated whether there are genotype-specific differences of the soy isoflavone response in asthma clinical outcomes in the SOYA population. Briefly, participants were randomly assigned in a 1:1 allocation ratio to receive either a soy isoflavone supplement or a matching placebo twice daily for 6 months. Race and ethnicity were self-reported by participants at baseline and at each spirometric test. Briefly, inclusion criteria were age 12 years or older, a physician's diagnosis of asthma, evidence of at least a 12% increase in FEV₁ after inhaling albuterol or a positive methacholine challenge result (20% decrease in FEV₁ at <16 mg/mL), FEV₁ equal to or greater than 50% of predicted prebronchodilator value, currently prescribed daily controller asthma medications, and evidence of poor asthma control. Poor asthma control was defined as having 1 or more of the following: a score of 19 or less on the Asthma Control Test (ACT), use of b-agonist for asthma symptoms 2 or more times per week, nocturnal awakening with asthma symptoms more than once per week, and 2 or more episodes of asthma exacerbations in the past 12 months.

The total number of participants in the original study was 386 (placebo, 193; soy isoflavones, 193). Using the remaining DNA, the inventors were able to genotype 120 patients treated with soy isoflavones and 145 placebo-treated control subjects (total = 265, Table I). These 265 subjects were included in the study. The inventors chose to use the 4G/5G single nucleotide polymorphisms (SNPs; rs1799768) rather than the A/G SNP (rs2227361) because the 4G/5G SNP has been well studied, including functional studies with DNA promotor constructs. The study population was relatively small (making a genome-wide association study, which the inventors used in a previous study of the rs22227631 SNP, impractical), there was a high degree of linkage disequilibrium between the 2 SNPs, and rs22227631 was commonly used as a surrogate marker of rs1799768. This study was conducted with approval of the Institutional Review Boards at Northwestern University and the University of South Florida.

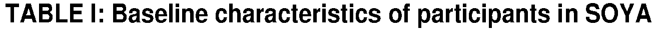

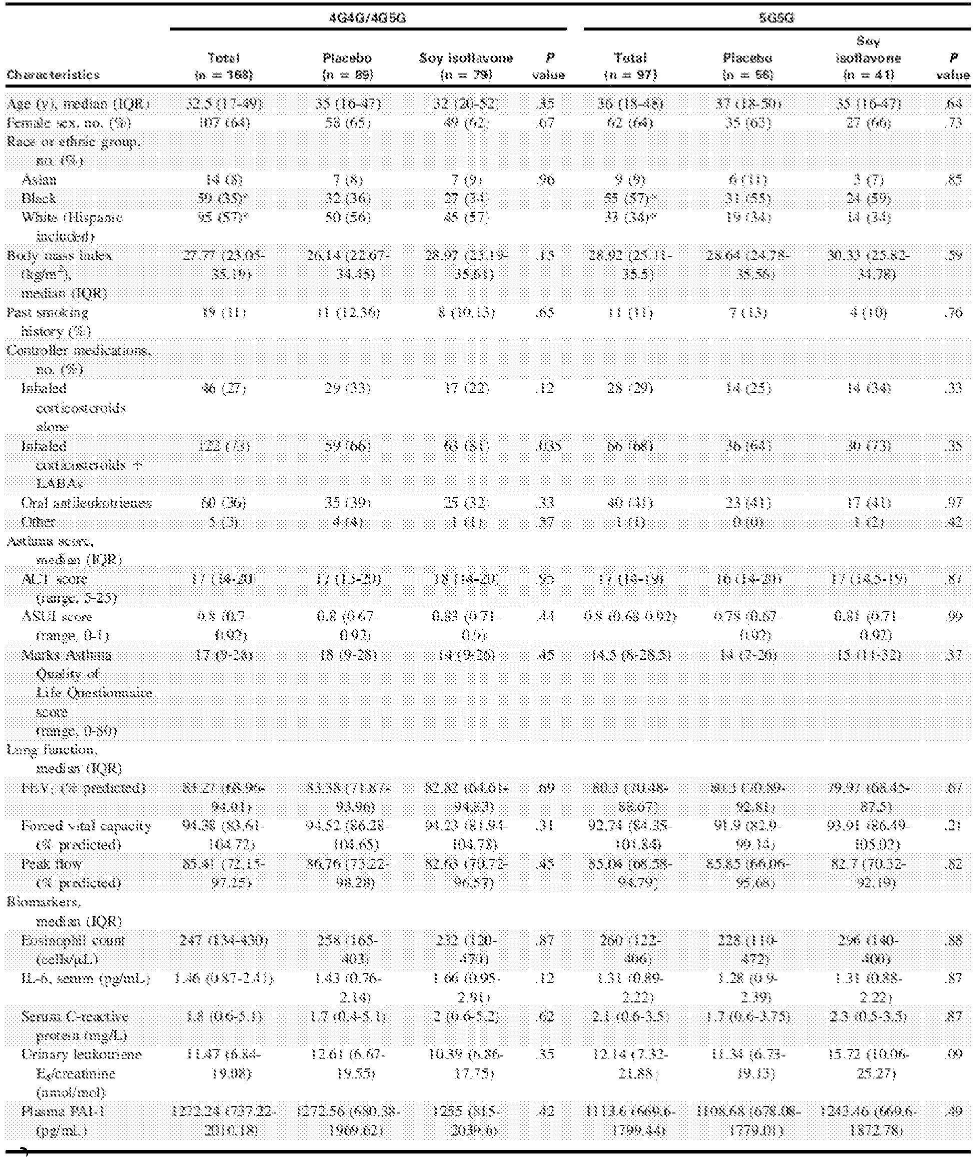

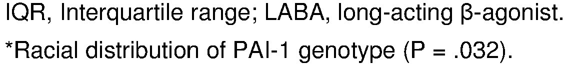

### Outcome Measures

Based on the hypothesis of PAI-1 gene-environment interaction on asthma control, the primary outcome measure was asthma exacerbation. Rates of episodes of asthma exacerbation were defined from diary cards by 1 or more of the following: 30% or greater decrease in morning peak expiratory flow (from personal best) for 2 consecutive days, addition of oral corticosteroids to treat asthma symptoms, unscheduled contact with a health care practitioner (emergency department, physician office, and hospital) for asthma symptoms, and increased use of rescue bronchodilator by 4 or more puffs of a metered-dose inhaler or 2 or more nebulizer treatments in 1 day. Secondary outcomes included symptom-free days, ACT score (range, 5-25), Asthma Symptoms Utility Index (ASUI) score (range, 0-1.0), and Marks Asthma Quality of Life questionnaire score (range, 0-80). Other outcomes included lung function, peak expiratory flow, exhaled nitric oxide values, peripheral blood eosinophil counts, and serum IL-6, serum C-reactive protein, urinary leukotriene E₄, and total blood genistein levels.

### PAI-1 Genotyping

Genomic DNA was extracted with the QIAamp kit (Qiagen, Valencia, Calif), and 4G5G genotyping was performed by using an allele-specific PCR with an alternative forward primer: GTCTGGACACGTGGGGG for the 5G allele (SEQ ID NO: 1) or GTCTGGACACGTGGGGA for the 4G allele (SEQ ID NO: 2), a common reverse primer: TGCAGCCAGCCACGTGATTGTCTAG (SEQ ID NO: 3), and a control upstream primer, AAGCTTTTACC ATGGTAACCCCTGGT (SEQ ID NO: 4), as previously described.

### Primary Bronchial Epithelial Cell Culture

Normal human bronchial epithelial (NHBE) cells were cultured, as previously described in Cho SH, Lee SH, Kato A, Takabayashi T, Kulka M, Shin SC, et al. Cross-talk between human mast cells and bronchial epithelial cells in plasminogen activator inhibitor-1 production via transforming growth factor-b1. Am J Respir Cell Mol Biol 2015;52:88-95. NHBE cells were stimulated with recombinant human TGF-b1 (2 ng/mL; R&D Systems, Minneapolis, Minn) with and without genistein pretreatment (1 or 5 mmol/L; Sigma-Aldrich, St Louis, Mo) for 1 hour. Cell pellets were used for real-time PCR, and cell-culture supernatants were used for PAI-1 protein ELISA.

### ELISA and Real-Time PCR for PAI-1

The PAI-1 concentration in the blood of subjects and supernatants of NHBE cells was measured in duplicate by means of ELISA (Assay Pro, St Charles, Mo), as previously described in Cho et al. 2015. For mRNA analysis of PAI-1 (Hs00167155_m1), quantitative real-time PCR was performed with the ViiA 7 Real-Time PCR System (Applied Biosystems, Foster City, Calif). Expression of glyceraldehyde-3-phosphate dehydrogenase (GAPDH; Hs02786624_g1) was used as an internal control for normalization. Cycling conditions were as follows: 50°C for 2 minutes (hold) for 1 cycle, 95°C for 10 minutes (hold) for 1 cycle, and 95°C for 15 seconds and 60°C for 1 minute (cycling) for 40 cycles. Relative gene expression was calculated by using the comparative 2^{-ΔΔCT} method.

### Statistical Analyses

The inventors used a multivariable logistic regression model to analyze the effect of soy isoflavones (reference 5 placebo), PAI-1 genotype (rs1799889, reference 5 5G5G), and interaction terms (soy 3 PAI-1 genotype) on a history of allergy and eczema. Next, the inventors used a general linear model to analyze the effect of soy isoflavones (reference 5 placebo), PAI-1 genotype (rs1799889, reference 5 5G5G), and interaction terms (soy 3 PAI-1 genotype) on lung function, asthma scores, and laboratory markers according to the mean change from baseline to 24 weeks. Also, differences in person-year rates were compared using OpenEpi for episodes of asthma exacerbation components: peak flow (30% decrease), oral steroid use, rescue medication use, and unscheduled contact with a health care practitioner. 95% Cls were computed from a mid-P exact test. The method used in the OpenEpi was the exact and maximum likelihood statistics applied from a Pascal program by David Martin. Finally, multivariable negative binominal regression analysis was performed to allow for overdispersion with random intercepts for clinics on cases with events of any exacerbation: peak flow increase, new oral steroid use, rescue medication use, and unscheduled contact by those with the PAI-1 genotype. In all the models, the inventors also adjusted for the effects for the following variables: age, sex, race, body mass index, medication used, baseline lung function, any ongoing exposure to environmental tobacco smoke, and past smoking. The significance level is .05, and the hypothesis test is 2-sided for all tests. Analyses were performed with OpenEpi and SAS software (version 9.4; SAS Institute, Cary, NC).

### Results

### Genotype-specific differences in baseline and clinical characteristics

Table I presents the distribution of selected characteristics for the overall study sample divided by the PAI-1 genotype and treatment status, which is placebo versus soy isoflavones. DNA samples from a total of 265 subjects were available and able to be genotyped. The mean age of the study population was 35.0 years, and the distribution of race was 48% white (including Hispanic), 43% black, and 9% Asian. The overall distribution of PAI-1 genotype rs1799889 of 4G4G, 4G5G, and 5G5G was 42 (16%), 126 (48%), and 97 (37%), respectively. Because of a high percentage of black subjects in the study population, the size of the 4G4G genotype was relatively small (16%). To maximize the power, the 4G4G and 4G5G genotypes (high PAI-1-producing genotypes) were combined and compared with the 5G5G group as a reference in all subsequent analyses. There were no significant genotype-specific differences in baseline and clinical characteristics. In both the placebo and soy isoflavone groups, the percentage of black subjects in the 5G5G genotype group was significantly higher compared with that of the white subjects (57% vs 34%, P = .032). In the baseline questionnaire the risk of eczema and allergies was significantly greater in the 4G4G/4G5G group compared with the 5G5G group (odds ratio, 2.57 [95% Cl, 1.19-5.54; P *=* .016] and 2.35 [95% Cl, 1.10-5.03; P = .028], respectively). (See Table II) Otherwise, there were no significant differences in asthma symptom scores, lung function, and other baseline characteristics between the 2 groups.

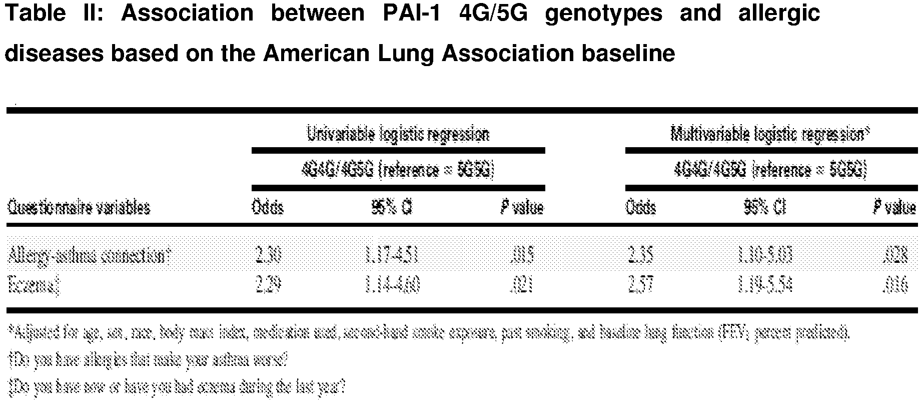

### Effect of genotype-specific differences in soy isoflavones on asthma exacerbation

The inventors first examined the asthma exacerbation measures. In multivariate negative binomial regression analyses, there were significantly more frequent asthma exacerbations, with new oral corticosteroid use in subjects with the 4G4G/4G5G genotype compared with the 5G5G genotype (incident rate ratio, 2.57; P = .031; Table III). Other components of asthma exacerbations were not significantly different between the 2 genotype groups. There was also a significant interaction between the PAI-1 genotype and soy isoflavone treatment only in the new use of oral corticosteroids (P = .005) but not in other asthma exacerbation components.

**Table III: Multivariable negative binomial regression analyses of PAI-1 genotype-soy isoflavone effect on asthma control**

| Asthma control outcomes | Multivariable negative binomial regression | | | | |
|---|---|---|---|---|---|
| | (Independent) variable | IRR | 95% CI | | P value |
| Any exacerbation | Soy (reference 5 placebo) | 1.30 | 0.78 | 2.17 | .31 |
| | 4G4G/4G5G (reference 5 5G5G) | 1.15 | 0.74 | 1.80 | .53 |
| | Genotype-soy interaction | | | | .17 |
| New use of oral steroids | Soy (reference 5 placebo) | 1.59 | 0.58 | 4.40 | .37 |
| | 4G4G/4G5G (reference 5 5G5G) | 2.57 | 1.09 | 6.07 | .031 |
| | Genotype-soy interaction | | | | .005 |
| Peak flow decrease (30%) | Soy, yes (reference 5 no) | 1.25 | 0.51 | 3.06 | .62 |
| | Group 4 (reference 5 5) | 0.73 | 0.33 | 1.61 | .43 |
| | Genotype-soy interaction | | | | .91 |
| Increased use of rescue medications | Soy (reference 5 placebo) | 1.40 | 0.66 | 2.99 | .38 |
| | 4G4G/4G5G (reference 5 5G5G) | 1.23 | 0.64 | 2.35 | .53 |
| | Genotype-soy interaction | | | | .25 |
| Unscheduled contact | Soy, yes (reference 5 no) | 1.29 | 0.40 | 4.16 | .67 |
| | Group 4 (reference 5 5) | 0.75 | 0.25 | 2.25 | .61 |
| | Genotype-soy interaction | | | | .33 |

| | | | | | |
|---|---|---|---|---|---|
| *IRR, Incident rate ratio.* **Adjusted for age, sex, race, body mass index, medication used, second-hand smoke exposure, past smoking, and baseline lung function (FEV₁ percent predicted)* | | | | | |

The inventors performed further PAI-1 genotype-specific subgroup negative binomial regression analyses on asthma exacerbation rates. (see Table IV). New oral corticosteroid use was significantly reduced by soy isoflavone treatment compared with placebo in subjects with the 4G4G/4G5G genotype (incident rate ratio, 0.22; *P* <.001) but not in those with the 5G5G genotype. There was a signal of unscheduled contact risk; however, there was no interaction between the PAI-1 genotype and soy isoflavone treatment in the unscheduled contact component (*P* = .33, Table II).

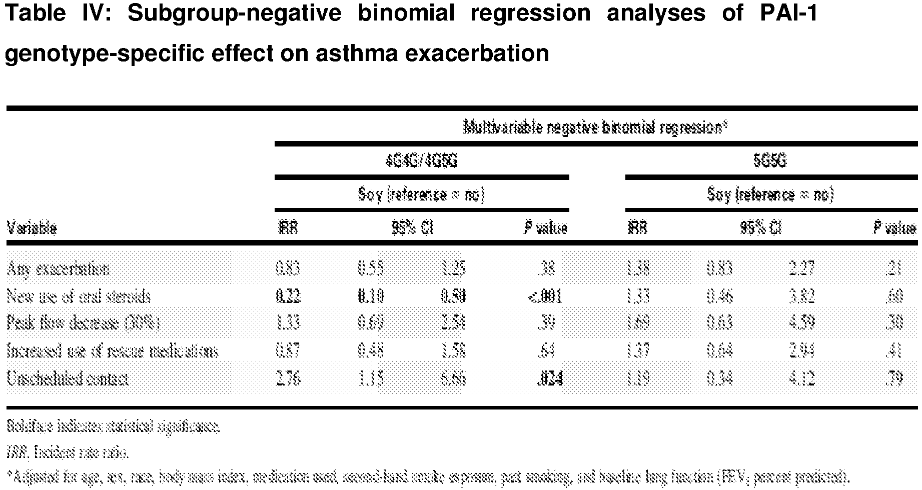

The inventors also measured the PAI-1 genotype-specific effect on the number of episodes of poor asthma control. There was no significant difference between the soy isoflavone and placebo treatment groups among the subjects with the 5G5G genotype (Table V). However, soy isoflavone treatment reduced new use of oral corticosteroids per person per year by 4-fold compared with the placebo control (0.2 vs 0.8; relative risk, 0.28 [95% Cl, 0.12-0.59; *P* < .001]) in the 4G4G/4G5G genotype group.

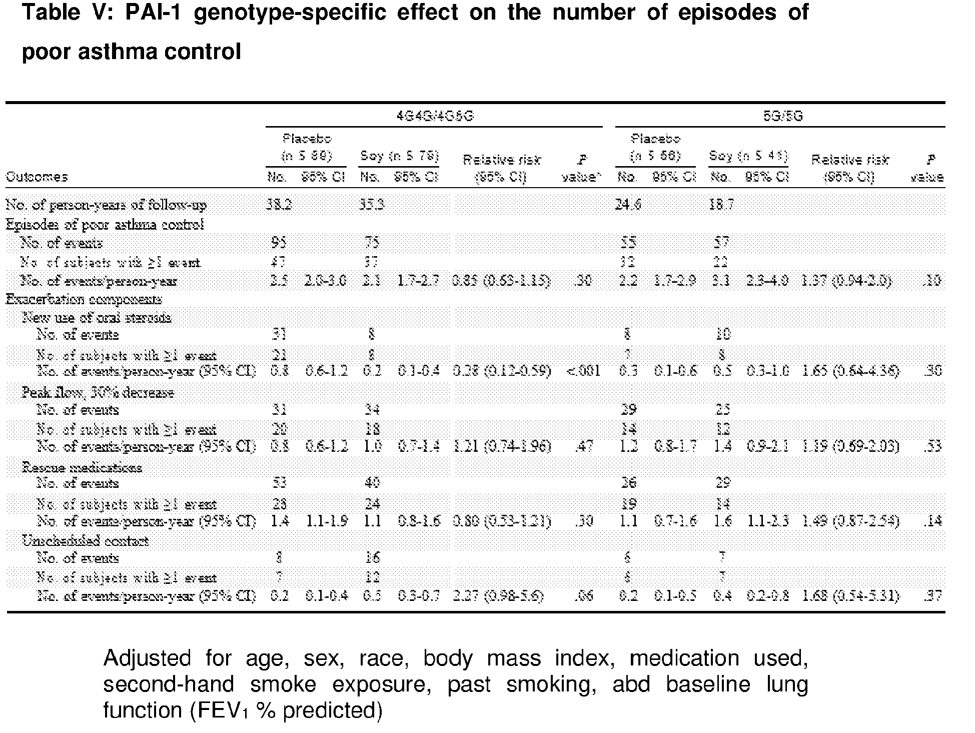

Other components of asthma exacerbation were not significantly different between the 2 treatment groups, although there was a trend of greater unscheduled contact (unscheduled contact with a health care practitioner in the emergency department, physician's office, or hospital) for asthma symptoms in the soy isoflavone group (0.5 vs 0.2; relative risk, 2.27; *P* = .06) compared with the placebo group. However, the total number of unscheduled contacts was 32, and only 4 incidents resulted in oral corticosteroid use, indicating that the unscheduled contact measure only infrequently represented a severe exacerbation.

To overcome the limitation of self-reported ethnic adjustment for the component of asthma exacerbation, the inventors performed a subgroup analysis based on the 2 major races: white (48%) and black (43%). It was found that soy isoflavones significantly reduced new use of oral steroids compared with placebo in the 4G4G/4G5G genotype groups both among white (3.7-fold, 0.2 vs. 0.7, *P* = .015) and black (3.2-fold, 0.3 vs 1.1, *P* = .029) participants. (Tables VI and VII)

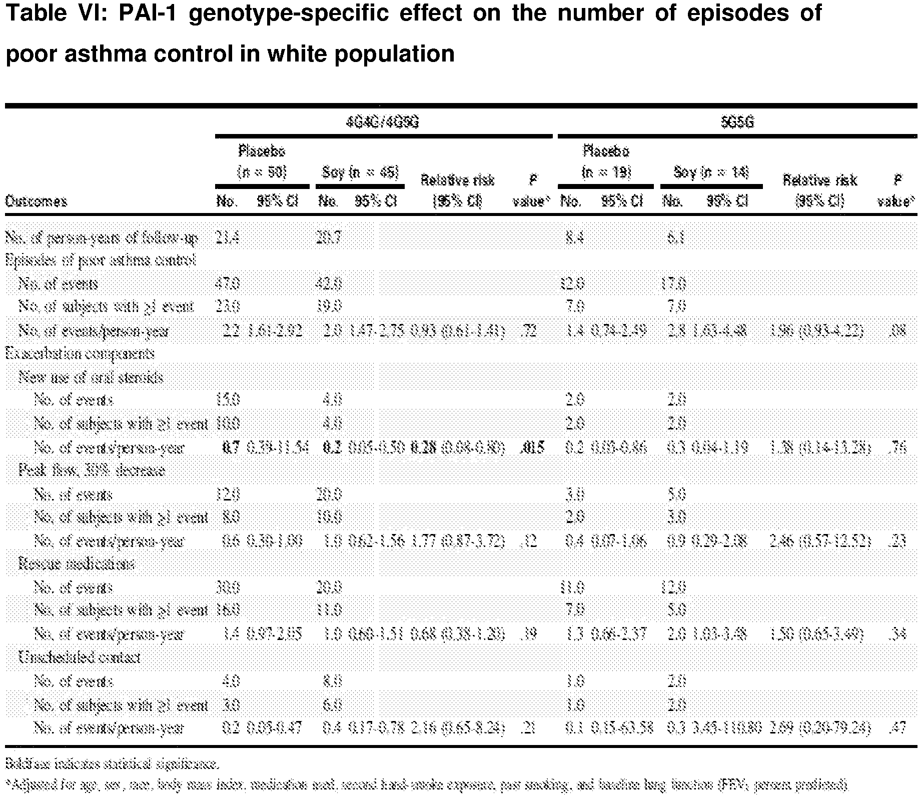

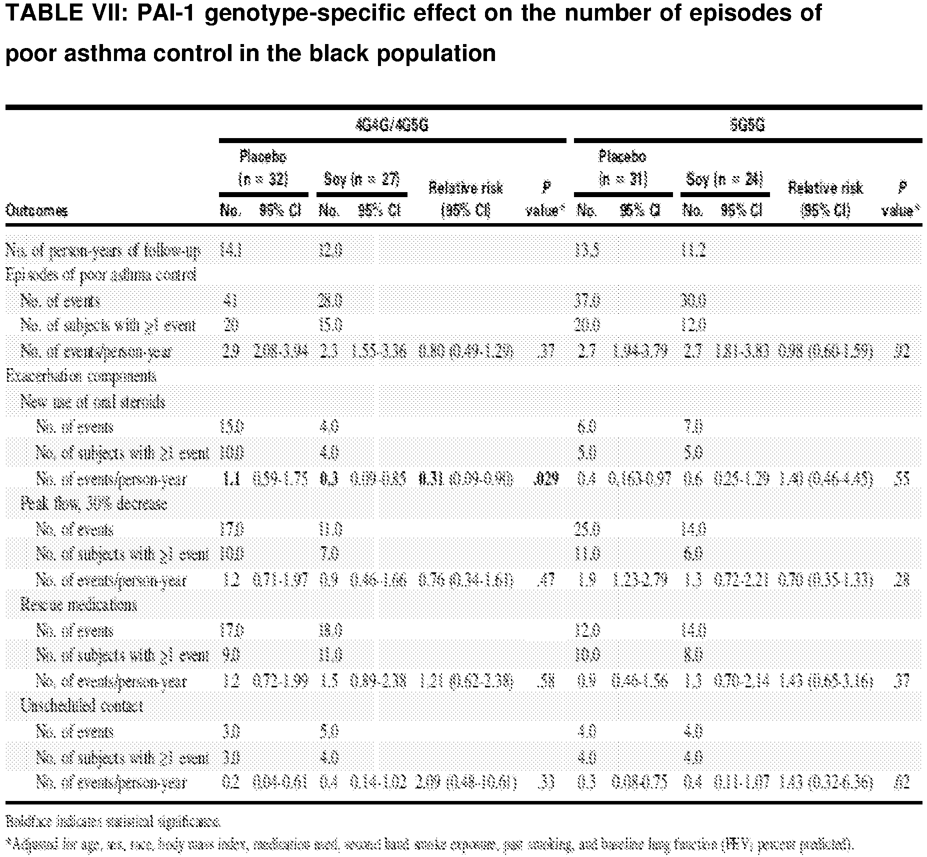

### Effect of genotype-specific differences in soy isoflavone on other clinical and laboratory outcomes

There was no significant genotype-specific effect of soy isoflavone treatment on lung function parameters, such as FEV₁ percent predicted or forced vital capacity percent predicted (Table VIII). However, there was a significant interaction between soy isoflavone treatment and genotype in improving peak flow percentage with soy isoflavone treatment in the 4G4G/4G5G group (*P =* .047). In the other clinical outcomes (ACT, ASUI, and Marks Asthma Quality of Life Questionnaire cores), there were no significant interactions between soy isoflavone treatment and genotype other than a minor improvement in ASUI score in the 5G5G group (*P* = .027). There were no significant genotype-specific changes by isoflavone treatment in inflammatory markers, such as blood eosinophil counts and IL-6, C-reactive protein, and urine leukotriene E₄ levels. As expected, genistein levels were significantly increased with soy isoflavone treatment in both the 4G4G/4G5G and 5G5G groups (207.47 and 327.23 ng/mL, *P* < .001), and there were no significant differences between the 2 genotype groups. Plasma levels of PAI-1 were significantly decreased with soy isoflavone treatment in both the 4G4G/4G5G and 5G5G groups (270.11 vs 2230.45 pg/mL, P = .051), but there were no significant differences between the 2 genotype groups. This significant decrease of plasma PAI-1 levels after soy isoflavone treatment suggests a biologic role of genistein in downregulation of PAI-1 in human subjects.

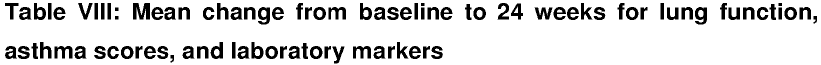

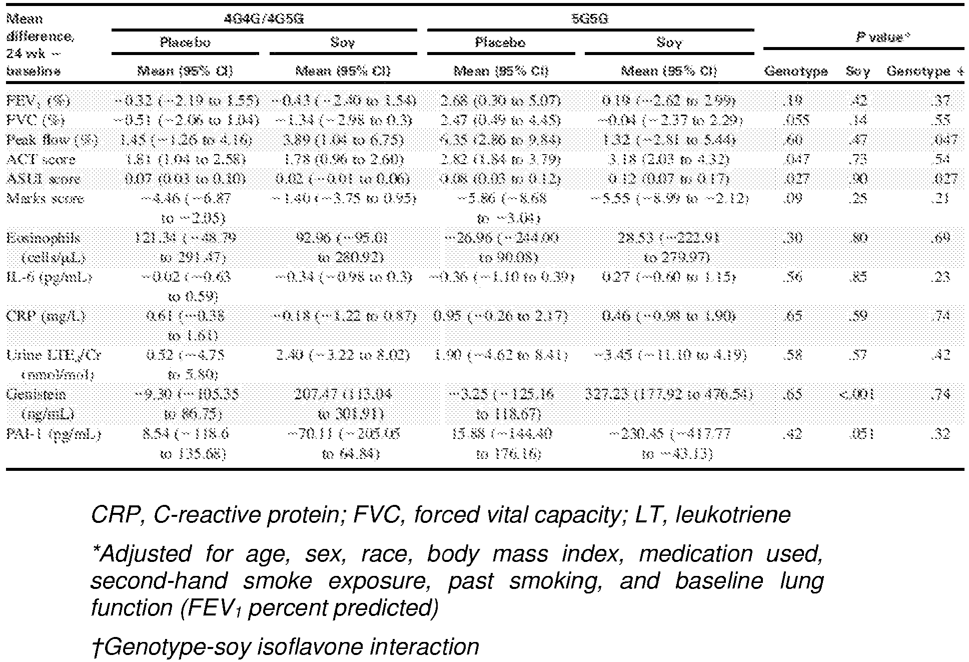

### Inhibition of PAI-1 production from airway epithelial cells by genistein

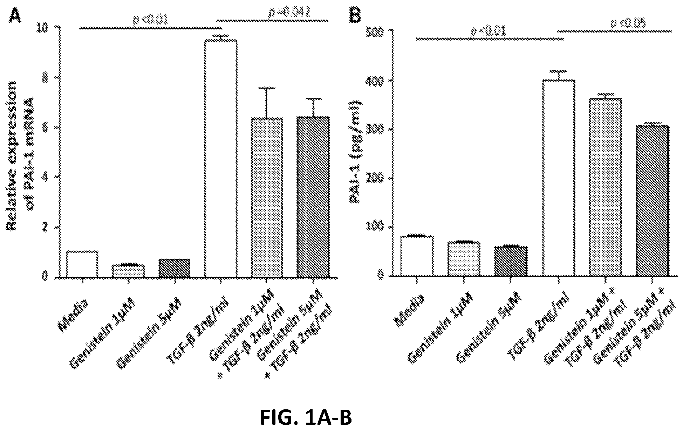

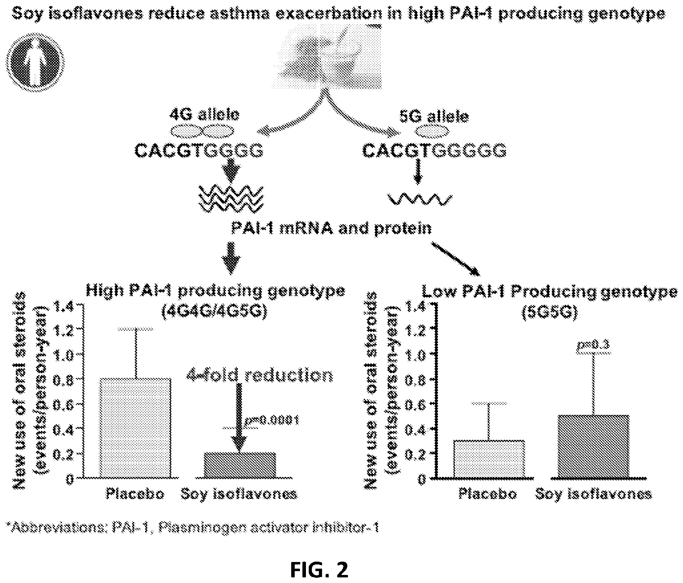

To investigate the cellular mechanism of the soy isoflavone/ PAI-1 link, the inventors treated cultured NHBE cells with TGF-β1 (2 ng/mL) for 6 hours with and without genistein pretreatment. There was a significant increase in PAI-1 mRNA and protein production (about 10-fold) after adding TGF-β1 (Fig 1). Genistein treatment significantly reduced TGF-β1-induced PAI-1 gene expression and protein production in a dose- dependent manner (360.7 ± 10.5 ng/mL [1 mmol/L] and 306.0 ± 5.3 pg/mL [5 mmol/L] vs 393.3 ± 20.7 ng/mL [no treatment], P < .05). This suggests that genistein can reduce upregulated PAI-1 production in the airways of asthmatic patients. Soy isoflavones reduce asthma exacerbation in high PAI-1 producing genotype. (Fig. 2)

### Conclusion

Soy isoflavone treatment has a significant benefit in reducing the number of severe asthma exacerbations in asthmatic patients with the high PAI-1-producing genotypes, suggesting that PAI-1 polymorphisms are a genetic biomarker for soy isoflavone- responsive asthma. Genistein-induced reduction of PAI-1 generation from airway epithelial cells might be part of the underlying therapeutic mechanism. These data stress the importance of precision medicine beyond simple phenotypic/endotypic approaches based on inflammatory subtypes, such as eosinophilic subtype.

### Example 2 - Soy Isoflavones for Prevention of Asthma in Pediatric Patients at Risk for Asthma

### Multiple phenotypes of pediatric asthma and the need for a precision approach to prevention

Pediatric asthma affects up to 12% of U.S. children and accounts for 5.92 billion dollars of U.S. health care expenditure annually. (Mannino DM, Homa DM, Akinbami LJ, Moorman JE, Gwynn C, Redd SC. Surveillance for asthma--United States, 1980-1999. MMWR Surveill Summ 2002;51:1-13; Sullivan PW, Ghushchyan V, Navaratnam P, et al. The national cost of asthma among school-aged children in the United States. Annals of allergy, asthma & immunology : official publication of the American College of Allergy, Asthma, & Immunology 2017;119:246-52). Approximately 80% of the children who progress to have asthma will have wheeze in early childhood, suggesting that primary prevention should target infancy. (Yunginger JW, Reed CE, O'Connell EJ, Melton LJ, 3rd, O'Fallon WM, Silverstein MD. A community-based study of the epidemiology of asthma. Incidence rates, 1964-1983. Am Rev Respir Dis 1992;146:888-94). However, even in early childhood, there are different phenotypes with the high morbidity group of children in the URECA cohort equally divided between atopic and non-atopic phenotypes. (Bacharier LB, Beigelman A, Calatroni A, et al. Longitudinal Phenotypes of Respiratory Health in a High-Risk Urban Birth Cohort. Am J Respir Crit Care Med 2019;199:71-82). Thus, disease heterogeneity starts in early childhood, and there is a need for a precision medicine approach to prevention.

In particular, severe early life viral LRI's show strong associations with asthma development, with effects ranging from 2.6 to 9.8 fold increases in asthma risk for HRV and RSV respectively. (Bacharier LB, Beigelman A, Calatroni A, et al. Longitudinal Phenotypes of Respiratory Health in a High-Risk Urban Birth Cohort. Am J Respir Crit Care Med 2019;199:71-82; Cal 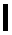 kan M, Bochkov YA, Kreiner-Møller E, et al. Rhinovirus wheezing illness and genetic risk of childhood-onset asthma. N Engl J Med 2013;368:1398-407; Jackson DJ, Gangnon RE, Evans MD, et al. Wheezing rhinovirus illnesses in early life predict asthma development in high-risk children. Am J Respir Crit Care Med 2008;178:667-72; Lemanske RF, Jr., Jackson DJ, Gangnon RE, et al. Rhinovirus illnesses during infancy predict subsequent childhood wheezing. The Journal of allergy and clinical immunology 2005;116:571-7; Liu AH, Babineau DC, Krouse RZ, et al. Pathways through which asthma risk factors contribute to asthma severity in inner-city children. The Journal of allergy and clinical immunology 2016;138:1042-50; Sigurs N, Gustafsson PM, Bjarnason R, et al. Severe respiratory syncytial virus bronchiolitis in infancy and asthma and allergy at age 13. Am J Respir Crit Care Med 2005;171:137-41; Kusel MM, de Klerk NH, Kebadze T, et al. Early-life respiratory viral infections, atopic sensitization, and risk of subsequent development of persistent asthma. The Journal of allergy and clinical immunology 2007;119:1105-10).

It has been shown that the risk of asthma development after viral illness is modified by host genetic predisposition. (Cal 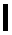 kan M, Bochkov YA, Kreiner-Møller E, et al. Rhinovirus wheezing illness and genetic risk of childhood-onset asthma. N Engl J Med 2013;368:1398-407; Cho SH, Min JY, Kim DY, et al. Association of a PAI-1 Gene Polymorphism and Early Life Infections with Asthma Risk, Exacerbations, and Reduced Lung Function. PloS one 2016;11:e0157848). Specifically, the inventors have studied a gain of function PAI-1 promoter variant with a single guanosine insertion/deletion variation at position -675 in the promoter region of the PAI-1 gene (rs1799768, 4G or 5G). (Cho SH, Min JY, Kim DY, et al. Association of a PAI-1 Gene Polymorphism and Early Life Infections with Asthma Risk, Exacerbations, and Reduced Lung Function. PloS one 2016;11:e0157848; Cho S, Kang J, Lyttle C, et al. Association of elevated plasminogen activator inhibitor 1 levels with diminished lung function in patients with asthma. Annals of allergy, asthma & immunology : official publication of the American College of Allergy, Asthma, & Immunology 2011;106:371-7; Cho SH, Hong SJ, Chen H, et al. Plasminogen activator inhibitor-1 in sputum and nasal lavage fluids increases in asthmatic patients during common colds. The Journal of allergy and clinical immunology 2014;133:1465-7, 7 e1-2; Cho SH, Jo A, Casale T, et al. Soy isoflavones reduce asthma exacerbation in asthmatic patients with high PAI-1-producing genotypes. The Journal of allergy and clinical immunology 2019; Cho SH, Tam SW, Demissie-Sanders S, Filler SA, Oh CK. Production of plasminogen activator inhibitor-1 by human mast cells and its possible role in asthma. Journal of immunology 2000;165:3154-61; Sherenian MG, Cho SH, Levin A, et al. PAI-1 gain-of-function genotype, factors increasing PAI-1 levels, and airway obstruction: The GALA II Cohort. Clinical and experimental allergy : journal of the British Society for Allergy and Clinical Immunology 2017;47:1150-8).

### PAI-1 promoter variation is frequent and affects both established asthma and increases the risk of developing asthma in the context of viral illnesses up to 17 fold

If children with ≥1 copy of the PAI-1 risk allele had a respiratory viral illness requiring a MD visit before 2 years old, these subjects had a 12-fold (any virus) to 18 fold (RSV) increased risk of developing asthma, with a significant gene by virus interaction. (Cho SH, Min JY, Kim DY, et al. Association of a PAI-1 Gene Polymorphism and Early Life Infections with Asthma Risk, Exacerbations, and Reduced Lung Function. PloS one 2016;11:e0157848). This finding is important because the genotype is common, present in homozygote or heterozygote form in 60% of the populations the inventors studied including asthmatic minority populations and the SOYA clinical trial. (Cho SH, Min JY, Kim DY, et al. Association of a PAI-1 Gene Polymorphism and Early Life Infections with Asthma Risk, Exacerbations, and Reduced Lung Function. PloS one 2016;11:e0157848; Cho S, Kang J, Lyttle C, et al. Association of elevated plasminogen activator inhibitor 1 levels with diminished lung function in patients with asthma. Annals of allergy, asthma & immunology : official publication of the American College of Allergy, Asthma, & Immunology 2011;106:371-7; Sherenian MG, Cho SH, Levin A, et al. PAI-1 gain-of-function genotype, factors increasing PAI-1 levels, and airway obstruction: The GALA II Cohort. Clinical and experimental allergy : journal of the British Society for Allergy and Clinical Immunology 2017;47:1150-8; Smith LJ, Kalhan R, Wise RA, et al. Effect of a soy isoflavone supplement on lung function and clinical outcomes in patients with poorly controlled asthma: a randomized clinical trial. JAMA 2015;313:2033-43).

The genotype is functional, constitutively increasing transcription of PAI-1, with associated higher levels. (Cho SH, Min JY, Kim DY, et al. Association of a PAI-1 Gene Polymorphism and Early Life Infections with Asthma Risk, Exacerbations, and Reduced Lung Function. PloS one 2016;11:e0157848; Cho SH, Jo A, Casale T, et al. Soy isoflavones reduce asthma exacerbation in asthmatic patients with high PAI-1-producing genotypes. The Journal of allergy and clinical immunology 2019; Sherenian MG, Cho SH, Levin A, et al. PAI-1 gain-of-function genotype, factors increasing PAI-1 levels, and airway obstruction: The GALA II Cohort. Clinical and experimental allergy : journal of the British Society for Allergy and Clinical Immunology 2017;47:1150-8). In addition to increasing asthma risk, the inventors found that higher plasma PAI-1 levels were associated with lower FEV₁ and FVC and the polymorphism was associated with decreased lung function in asthmatic subjects and a 2.6 fold increased risk of severe asthma exacerbations (Incident rate ratio 2.57, p=0.03). ( Cho S, Kang J, Lyttle C, et al. Association of elevated plasminogen activator inhibitor 1 levels with diminished lung function in patients with asthma. Annals of allergy, asthma & immunology : official publication of the American College of Allergy, Asthma, & Immunology 2011;106:371-7; Sherenian MG, Cho SH, Levin A, et al. PAI-1 gain-of-function genotype, factors increasing PAI-1 levels, and airway obstruction: The GALA II Cohort. Clinical and experimental allergy : journal of the British Society for Allergy and Clinical Immunology 2017;47:1150-8; Cho SH, Jo A, Casale T, et al. Soy isoflavones reduce asthma exacerbation in asthmatic patients with high PAI-1-producing genotypes. The Journal of allergy and clinical immunology 2019).

*PAI-1 promotes a Th2 airway endotype at the time of viral illness via TSLP*

PAI-1 in vitro enhanced TSLP production from normal human bronchial airway epithelial cells in response to IL-4/dsRNA TLR3 stimulation, which mimics the effects of a viral insult in an allergic airway. (Jo A, Lee SH, Kim DY, et al. Mast cell-derived plasminogen activator inhibitor type 1 promotes airway inflammation and remodeling in a murine model of asthma. The Journal of allergy and clinical immunology 2018;142:294-7). The inventors have previously shown that sputum levels of PAI-1 are significantly elevated in asthmatic subjects at baseline, and are further elevated during the peak of cold symptoms. (Cho SH, Hong SJ, Chen H, et al. Plasminogen activator inhibitor-1 in sputum and nasal lavage fluids increases in asthmatic patients during common colds. The Journal of allergy and clinical immunology 2014;133:1465-7, 7 e1-2). TSLP is a critical innate immune cytokine, inducing the production of Th2 cytokines such as IL-4 and IL-5 which in turn may perpetuate local IgE production, eosinophilic inflammation, and further airway remodeling. (Ziegler SF, Roan F, Bell BD, Stoklasek TA, Kitajima M, Han H. The biology of thymic stromal lymphopoietin (TSLP). Advances in pharmacology 2013;66:129-55). PAI-1 inhibition reduces eosinophilic inflammation, decreases airway remodeling, reduces local allergen-specific IgE production and the levels of IL-4 and IL-5. (Lee SH, Eren M, Vaughan DE, Schleimer RP, Cho SH. A plasminogen activator inhibitor-1 inhibitor reduces airway remodeling in a murine model of chronic asthma. American journal of respiratory cell and molecular biology 2012;46:842-6; Miyamoto S, Hattori N, Senoo T, et al. Intra-airway administration of small interfering RNA targeting plasminogen activator inhibitor-1 attenuates allergic asthma in mice. American journal of physiology Lung cellular and molecular physiology 2011;301:L908-16; Liu RM, Eldridge S, Watanabe N, et al. Therapeutic potential of an orally effective small molecule inhibitor of plasminogen activator inhibitor for asthma. American journal of physiology Lung cellular and molecular physiology 2016;310:L328-36).

### PAI-1 associated increases in local IgE production and T2 airway inflammation may decrease antiviral interferon responses of pDC's in the airway via increased FcεRI expression

PAI-1 increases local IgE production and levels of IL-4 and IL-5. (Liu RM, Eldridge S, Watanabe N, et al. Therapeutic potential of an orally effective small molecule inhibitor of plasminogen activator inhibitor for asthma. American journal of physiology Lung cellular and molecular physiology 2016;310:L328-36). The presence of IgE stabilizes FcεRI expressions on the cell surface with associated increases in FcεRI on mast cells, basophils, and dendritic cells. (Kubota T, Mukai K, Minegishi Y, Karasuyama H. Different stabilities of the structurally related receptors for IgE and IgG on the cell surface are determined by length of the stalk region in their alpha-chains. Journal of immunology 2006;176:7008-14; Macglashan D, Jr. IgE and Fc{epsilon}RI regulation. Annals of the New York Academy of Sciences 2005;1050:73-88; Sihra BS, Kon OM, Grant JA, Kay AB. Expression of high-affinity IgE receptors (Fc epsilon RI) on peripheral blood basophils, monocytes, and eosinophils in atopic and nonatopic subjects: relationship to total serum IgE concentrations. The Journal of allergy and clinical immunology 1997;99:699-706; Dehlink E, Baker AH, Yen E, Nurko S, Fiebiger E. Relationships between levels of serum IgE, cell-bound IgE, and IgE-receptors on peripheral blood cells in a pediatric population. PloS one 2010;5:e12204).

Notably, pDCs are a major source of type I interferon responses and cross linking of FcεRI on pDCs results in reduced type I interferon signaling. (Colonna M, Krug A, Cella M. Interferon-producing cells: on the front line in immune responses against pathogens. Current opinion in immunology 2002;14:373-9; Durrani SR, Montville DJ, Pratt AS, et al. Innate immune responses to rhinovirus are reduced by the high-affinity IgE receptor in allergic asthmatic children. The Journal of allergy and clinical immunology 2012;130:489-95; Gill MA, Bajwa G, George TA, et al. Counterregulation between the FcεRI pathway and antiviral responses in human plasmacytoid dendritic cells. Journal of immunology 2010;184:5999-6006; Gill MA, Liu AH, Calatroni A, et al. Enhanced plasmacytoid dendritic cell antiviral responses after omalizumab. The Journal of allergy and clinical immunology 2018;141 :1735-43.e9). On the other hand, both ex vivo and in vivo treatment with omalizumab restored interferon alpha production from pDCs, potentially due to decreased expression of FcεRIα. (Durrani SR, Montville DJ, Pratt AS, et al. Innate immune responses to rhinovirus are reduced by the high-affinity IgE receptor in allergic asthmatic children. The Journal of allergy and clinical immunology 2012;130:489-95; Gill MA, Bajwa G, George TA, et al. Counterregulation between the FcεRI pathway and antiviral responses in human plasmacytoid dendritic cells. Journal of immunology 2010;184:5999-6006; Gill MA, Liu AH, Calatroni A, et al. Enhanced plasmacytoid dendritic cell antiviral responses after omalizumab. The Journal of allergy and clinical immunology 2018;141:1735-43.e9; Teach SJ, Gill MA, Togias A, et al. Preseasonal treatment with either omalizumab or an inhaled corticosteroid boost to prevent fall asthma exacerbations. The Journal of allergy and clinical immunology 2015;136:1476-85; Prussin C, Griffith DT, Boesel KM, Lin H, Foster B, Casale TB. Omalizumab treatment downregulates dendritic cell FcεRI expression. The Journal of allergy and clinical immunology 2003;112:1147-54). Decreasing PAI-1 and local IgE production may also impact antiviral responses via FcεRIα.

### Soy isoflavones decrease production of PAI-1 both systemically and in the airway, and are a potential intervention

In vitro and animal studies have reported an association between soy intake and changes in PAI-1 levels. (Nagasawa A, Fukui K, Kojima M, et al. Divergent effects of soy protein diet on the expression of adipocytokines. Biochemical and biophysical research communications 2003;311:909-14; van Hinsbergh VW, Vermeer M, Koolwijk P, Grimbergen J, Kooistra T. Genistein reduces tumor necrosis factor alpha-induced plasminogen activator inhibitor-1 transcription but not urokinase expression in human endothelial cells. Blood 1994;84:2984-91). The downregulation of TGFβ1-induced PAI-1 production from normal bronchial epithelial cells by genistein treatment suggests that genistein may reduce PAI-1 production in the airways of asthmatics. (Cho SH, Jo A, Casale T, et al. Soy isoflavones reduce asthma exacerbation in asthmatic patients with high PAI-1-producing genotypes. The Journal of allergy and clinical immunology 2019). Systemic levels of PAI-1 also significantly decreased in subjects taking soy genistein in the SOYA study. (Smith LJ, Kalhan R, Wise RA, et al. Effect of a soy isoflavone supplement on lung function and clinical outcomes in patients with poorly controlled asthma: a randomized clinical trial. JAMA 2015;313:2033-43). Thus soy isoflavones have the capacity to prevent a Th2 shift of the airway and restore antiviral responses by decreasing airway PAI-1 levels.

### Soy isoflavones are present in high doses in Soy Formulas and there is a good safety profile

The doses of soy genistein available in soy formula are greater than that given in the SOYA study. The total amount of active genistein in aglycone form in soy fed infants is estimated to range from 1.3-6.2 mg/kg body weight/day compared to the SOYA study with 1-2 mg/kg/day dosing. The corresponding plasma genistein level in soy fed infants is 757 ng/ml in contrast to the levels post supplementation in the SOYA study (37.767 ng/ml). While this level is greater than that given in the prior SOYA study in adults, these doses have not been associated with a significant toxicity profile. Ingestion of soy isoflavones has not been associated with changes in thelarche, pubarche, cycle length, fertility or male reproductive effects including alterations in sex organ development or sexual maturation. (Andres A, Moore MB, Linam LE, Casey PH, Cleves MA, Badger TM. Compared with feeding infants breast milk or cow-milk formula, soy formula feeding does not affect subsequent reproductive organ size at 5 years of age. The Journal of nutrition 2015;145:871-5; Duitama SM, Zurita J, Cordoba D, Duran P, Ilag L, Mejia W. Soy protein supplement intake for 12 months has no effect on sexual maturation and may improve nutritional status in pre-pubertal children. Journal of paediatrics and child health 2018;54:997-1004; Gilchrist JM, Moore MB, Andres A, Estroff JA, Badger TM. Ultrasonographic patterns of reproductive organs in infants fed soy formula: comparisons to infants fed breast milk and milk formula. The Journal of pediatrics 2010;156:215-20; Mendez MA, Anthony MS, Arab L. Soy-based formulae and infant growth and development: a review. The Journal of nutrition 2002;132:2127-30; Messina M, Rogero MM, Fisberg M, Waitzberg D. Health impact of childhood and adolescent soy consumption. Nutrition reviews 2017;75:500-15; Segovia-Siapco G, Pribis P, Oda K, Sabate J. Soy isoflavone consumption and age at pubarche in adolescent males. European journal of nutrition 2018;57:2287-94; Strom BL, Schinnar R, Ziegler EE, et al. Exposure to soy-based formula in infancy and endocrinological and reproductive outcomes in young adulthood. Jama 2001;286:807-14). While there was a report of advancement of menarche, this was only advanced by approximately 4.8 months. However, infants with congenital hypothyroidism may have a longer time for TSH normalization on replacement therapy, which is why this remains an exclusion. (Conrad SC, Chiu H, Silverman BL. Soy formula complicates management of congenital hypothyroidism. Archives of disease in childhood 2004;89:37-40).

### Prior Prevention studies

Heterogeneity of host factors may explain uneven findings in prior prevention studies which have utilized ICS, probiotics, and vitamin D. (Guilbert TW, Morgan WJ, Zeiger RS, et al. Long-term inhaled corticosteroids in preschool children at high risk for asthma. N Engl J Med 2006;354:1985-97; Mennini M, Dahdah L, Artesani MC, Fiocchi A, Martelli A. Probiotics in Asthma and Allergy Prevention. Front Pediatr 2017;5:165; West CE, Jenmalm MC, Kozyrskyj AL, Prescott SL. Probiotics for treatment and primary prevention of allergic diseases and asthma: looking back and moving forward. Expert Rev Clin Immunol 2016;12:625-39; Anderson LN, Chen Y, Omand JA, et al. Vitamin D exposure during pregnancy, but not early childhood, is associated with risk of childhood wheezing. Journal of developmental origins of health and disease 2015;6:308-16; Brustad N, Eliasen AU, Stokholm J, Bønnelykke K, Bisgaard H, Chawes BL. High-Dose Vitamin D Supplementation During Pregnancy and Asthma in Offspring at the Age of 6 Years. Jama 2019;321:1003-5; Litonjua AA, Carey VJ, Laranjo N, et al. Six-Year Follow-up of a Trial of Antenatal Vitamin D for Asthma Reduction. N Engl J Med 2020;382:525-33; Shen SY, Xiao WQ, Lu JH, et al. Early life vitamin D status and asthma and wheeze: a systematic review and meta-analysis. BMC pulmonary medicine 2018;18:120). Up to now, no asthma prevention studies have been carried out in a genotype specific manner.

The inventors find that PAI-1 promoter gain of function polymorphisms provide an opportunity to impact asthma development in a genotype specific manner in up to 60% of the population. Much of the prior work on PAI-1 has focused on the role of PAI-1 in airway remodeling. Plasminogen activator inhibitor-1 (PAI-1) promotes fibrosis and blocking this enzyme prevents extracellular matrix (ECM) deposition. (Eitzman DT, McCoy RD, Zheng X, et al. Bleomycin-induced pulmonary fibrosis in transgenic mice that either lack or overexpress the murine plasminogen activator inhibitor-1 gene. The Journal of clinical investigation 1996;97:232-7; Hattori N, Degen JL, Sisson TH, et al. Bleomycin-induced pulmonary fibrosis in fibrinogen-null mice. The Journal of clinical investigation 2000;106:1341-50; Lee SH, Eren M, Vaughan DE, Schleimer RP, Cho SH. A plasminogen activator inhibitor-1 inhibitor reduces airway remodeling in a murine model of chronic asthma. American journal of respiratory cell and molecular biology 2012;46:842-6; Miyamoto S, Hattori N, Senoo T, et al. Intra-airway administration of small interfering RNA targeting plasminogen activator inhibitor-1 attenuates allergic asthma in mice. American journal of physiology Lung cellular and molecular physiology 2011;301:L908-16).

While these prior studies suggest that there may be a role in asthma related airway fibrosis, the preliminary data shows that PAI-1 also effects TSLP and therefore promotes Th2 type inflammation in the airway. The inventors have previously shown an increased risk of asthma in those who carried a risk allele and had a viral illness in the first years of life is based on epidemiologic data. Children with the risk genotype (homo or heterozygous) with a severe viral illness will have less PAI-1 in the airway, greater IFN-α production during viral illness, and ultimately have decreased risk of developing Th2 airway endotypes if treated with soy isoflavones during viral seasons.

### PAI-1 and development of T2-high inflammation in the airway associated with viral illness

Subjects with the gain of function polymorphism in the PAI-1 promoter have higher PAI-1 levels, worse lung function, worse asthma outcomes, and, if symptomatic with an early life respiratory viral illness, a dramatically increased risk of developing asthma. (Cho SH, Min JY, Kim DY, et al. Association of a PAI-1 Gene Polymorphism and Early Life Infections with Asthma Risk, Exacerbations, and Reduced Lung Function. PloS one 2016;11:e0157848; Cho SH, Jo A, Casale T, et al. Soy isoflavones reduce asthma exacerbation in asthmatic patients with high PAI-1-producing genotypes. The Journal of allergy and clinical immunology 2019; Sherenian MG, Cho SH, Levin A, et al. PAI-1 gain-of-function genotype, factors increasing PAI-1 levels, and airway obstruction: The GALA II Cohort. Clinical and experimental allergy : journal of the British Society for Allergy and Clinical Immunology 2017;47:1150-8). The mechanism which could explain the joint effect of genotype and viral illness on asthma development is not yet established.

PAI-1 inhibition reduces eosinophilic inflammation and decreases airway remodeling. (Lee SH, Eren M, Vaughan DE, Schleimer RP, Cho SH. A plasminogen activator inhibitor-1 inhibitor reduces airway remodeling in a murine model of chronic asthma. American journal of respiratory cell and molecular biology 2012;46:842-6; Miyamoto S, Hattori N, Senoo T, et al. Intra-airway administration of small interfering RNA targeting plasminogen activator inhibitor-1 attenuates allergic asthma in mice. American journal of physiology Lung cellular and molecular physiology 2011;301:L908-16). PAI-1 inhibition reduces local allergen-specific IgE production and the levels of IL-4 and IL-5. (Liu RM, Eldridge S, Watanabe N, et al. Therapeutic potential of an orally effective small molecule inhibitor of plasminogen activator inhibitor for asthma. American journal of physiology Lung cellular and molecular physiology 2016;310:L328-36). However, the mechanisms of this association between PAI-1 and T2/eosinophilic inflammation are not clearly understood.

TSLP is a critical innate immune cytokine in inducing the production of Th2 cytokines such as IL-4 and IL-5, which in turn may perpetuate local IgE production, eosinophilic inflammation, and further airway remodeling. (Ziegler SF, Roan F, Bell BD, Stoklasek TA, Kitajima M, Han H. The biology of thymic stromal lymphopoietin (TSLP). Advances in pharmacology 2013;66:129-55). The inventors recently found that PAI-1 enhances the dsRNA/IL-4-induced production of TSLP from airway EpCs. This suggests that viral insults in early life in genetically susceptible subjects may increase T2 high inflammation. Delineating airway endotypes both at the time of viral illness and after resolution of illness in subjects with this polymorphism is innovative, as prior studies which have evaluated airway responses have not stratified by genotype and may therefore been unable to identify these patterns. ( Jackson DJ, Makrinioti H, Rana BM, et al. IL-33-dependent type 2 inflammation during rhinovirus-induced asthma exacerbations in vivo. Am J Respir Crit Care Med 2014;190:1373-82; Thwaites RS, Coates M, Ito K, et al. Reduced Nasal Viral Load and IFN Responses in Infants with Respiratory Syncytial Virus Bronchiolitis and Respiratory Failure. Am J Respir Crit Care Med 2018;198:1074-84; Thwaites RS, Jarvis HC, Singh N, et al. Absorption of Nasal and Bronchial Fluids: Precision Sampling of the Human Respiratory Mucosa and Laboratory Processing of Samples. Journal of visualized experiments : JoVE 2018).

### PAI-1 and IFN-α responses to viral illness

The inventors have shown an association of the PAI-1 promoter genotype and IgE levels, corroborating prior findings. This is relevant since the level of IgE stabilizes FcεRI expressions on pDCs, a major source of type I interferon responses. (Dehlink E, Baker AH, Yen E, Nurko S, Fiebiger E. Relationships between levels of serum IgE, cell-bound IgE, and IgE-receptors on peripheral blood cells in a pediatric population. PloS one 2010;5:e12204; Colonna M, Krug A, Cella M. Interferon-producing cells: on the front line in immune responses against pathogens. Current opinion in immunology 2002;14:373-9).

While it is known that cross linking of FcεRI on pDCs reduces type I interferon signaling, and treatment with omalizumab restores interferon alpha production from pDCs, it is not known if the same level of effect is shown with soy isoflavone interruption of PAI-1 and hence IgE production. (Durrani SR, Montville DJ, Pratt AS, et al. Innate immune responses to rhinovirus are reduced by the high-affinity IgE receptor in allergic asthmatic children. The Journal of allergy and clinical immunology 2012;130:489-95; Gill MA, Bajwa G, George TA, et al. Counterregulation between the FcεRI pathway and antiviral responses in human plasmacytoid dendritic cells. Journal of immunology 2010;184:5999-6006; Gill MA, Liu AH, Calatroni A, et al. Enhanced plasmacytoid dendritic cell antiviral responses after omalizumab. The Journal of allergy and clinical immunology 2018;141:1735-43.e9). This therapy can affect allergic airway responses and also have impact on antiviral responses which is a novel area of research.

### PAI-1 promotor polymorphism in combination with early life viral illness increases asthma risk 17 fold

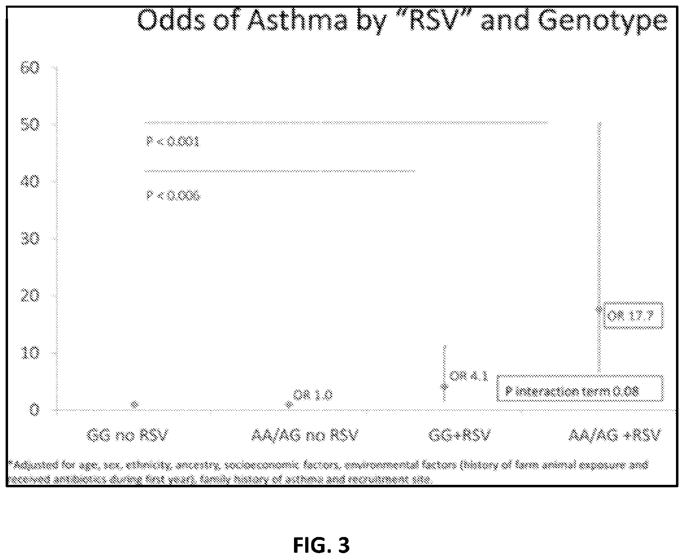

PAI-1 promoter polymorphism in combination with early life viral illness increases asthma risk 17 fold. In the GALA2 study, the inventors found that the combination of an early life viral illness requiring medical attention and the PAI-1 polymorphism had a joint effect on the risk of developing asthma. In those with the polymorphism, the risk of asthma ranged from 11.7 (any virus) to 17.7 fold increased risk (RSV) (Fig.3). To increase estimate precision and sample size, the inventors combined heterozygotes and homozygotes. However, there was a strong dose effect when heterozygotes (OR 15.5, 95% CI 4.7-51.3) and homozygotes (OR 24.9, 95%CI 3.2-194.1) were separated out.

### PAI-1 enhances the production of TSLP in TLR-3 agonist/IL-4-stimulated airway EpCs

TSLP is an alarmin which acts on DCs, decreasing production of Th1 pathway cytokines (including IL-12) and promoting the production of chemokines (eotaxin-2, IL-8, and TARC), which recruit eosinophils, neutrophils and Th2 cells to the airway. (Liu YJ. Thymic stromal lymphopoietin: master switch for allergic inflammation. The Journal of experimental medicine 2006;203:269-73; Rimoldi M, Chieppa M, Salucci V, et al. Intestinal immune homeostasis is regulated by the crosstalk between epithelial cells and dendritic cells. Nature immunology 2005;6:507-14). Thus TSLP acts to shift the airway towards a Th2 environment. (Papazian D, Hansen S, Wurtzen PA. Airway responses towards allergens - from the airway epithelium to T cells. Clinical and experimental allergy : journal of the British Society for Allergy and Clinical Immunology 2015;45:1268-87). TSLP is produced in the airway after both viral insult / TLR3 stimulation and exposure to Th2 cytokines. (Kato A, Favoreto S, Jr., Avila PC, Schleimer RP. TLR3- and Th2 cytokine-dependent production of thymic stromal lymphopoietin in human airway epithelial cells. Journal of immunology 2007;179:1080-7; Bogiatzi SI, Fernandez I, Bichet JC, et al. Cutting Edge: Proinflammatory and Th2 cytokines synergize to induce thymic stromal lymphopoietin production by human skin keratinocytes. Journal of immunology 2007;178:3373-7).

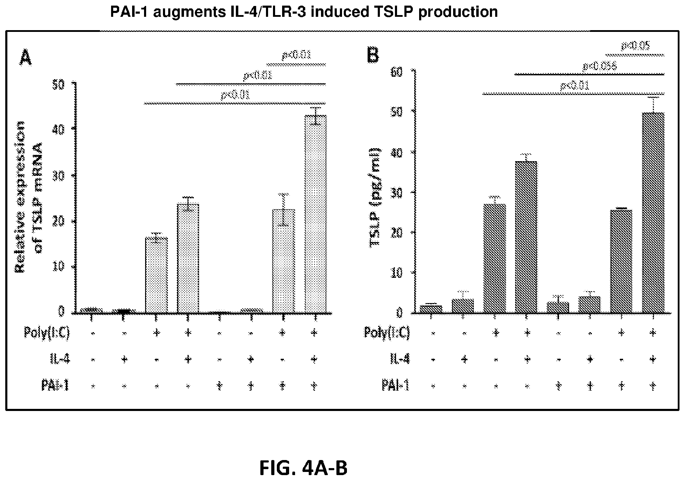

As noted in Figure 4, the inventors measured TSLP production (mRNA by RT-PCR of cells (A) and protein by ELISA of supernatant (B)) from normal bronchial epithelial cells after stimulation by IL-4/TLR-3 agonist (double stranded RNA (poly I:C)) with or without PAI-1 for 6 hours. PAI-1 dramatically enhanced the production of TSLP mRNA and protein in this model.

### Soy isoflavones and PAI-1 production

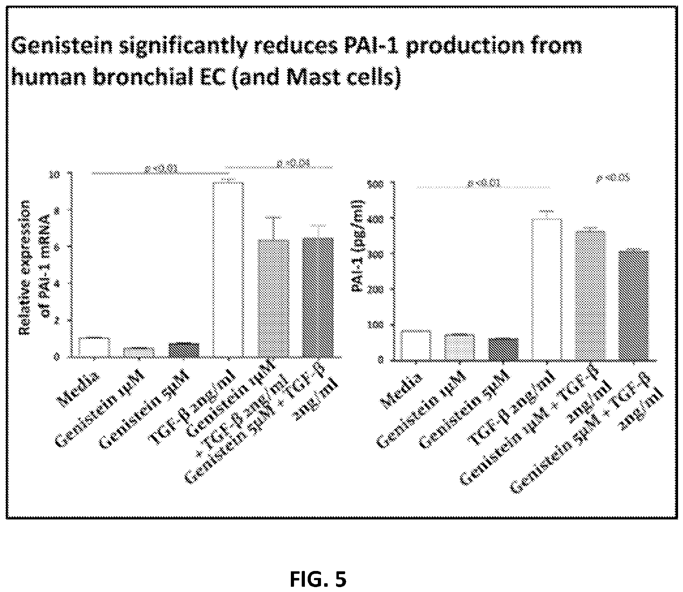

To demonstrate the soy isoflavone-PAl-1 link in vitro, the inventors cultured primary human bronchial epithelial cells (NHBE) and treated the cells with TGF-β1 (2ng/ml) for 6 hours with and without genistein pretreatment. There was a significant increase of the PAI-1 mRNA and protein production (about 10-fold) after adding the TGF-β1 (Fig 5). Genistein treatment significantly reduced TGF-β1-induced PAI-1 gene expression and protein production (360.7 ± 10.5ng/ml (1 µM) and 306.0 ± 5.3 pg/ml (5 µM) vs 393.3 ± 20.7 ng/ml (no treatment), p < 0.05). This result suggests that genistein reduces upregulated airway PAI-1 production in asthma.

### T2 endotypes and nasal epithelial transcriptomic signatures

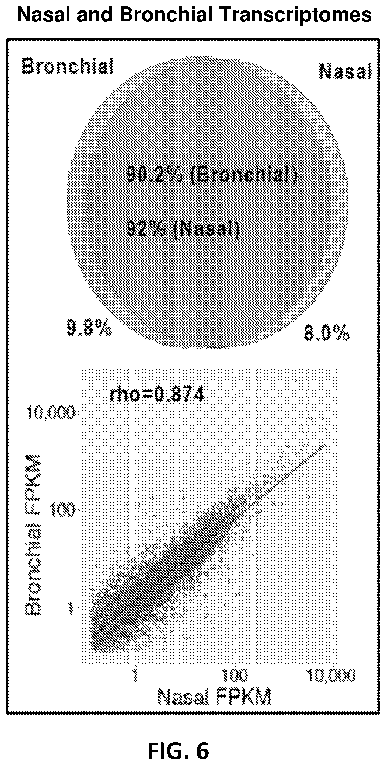

To determine if soy isoflavones influence airway endotype, a reliable marker of T2 inflammation is needed. IL-4, IL-5, IL-13 and TARC responses are canonically pronounced in atopic asthmatics with Th2 airway inflammation at baseline and at times of viral illness. (Jackson DJ, Makrinioti H, Rana BM, et al. IL-33-dependent type 2 inflammation during rhinovirus-induced asthma exacerbations in vivo. Am J Respir Crit Care Med 2014;190:1373-82; Hansel TT, Tunstall T, Trujillo-Torralbo MB, et al. A Comprehensive Evaluation of Nasal and Bronchial Cytokines and Chemokines Following Experimental Rhinovirus Infection in Allergic Asthma: Increased Interferons (IFN-gamma and IFN-lambda) and Type 2 Inflammation (IL-5 and IL-13). EBioMedicine 2017;19:128-38). However, protein levels including IL-4 and IL-5 have variability in both normal and asthmatic subjects' airway epithelium, resulting in difficulties in determining a cut point, which clearly delineates a T2 high phenotype. Another problem in evaluating airway endotypes in young children is the inability to directly sample the bronchial epithelium. To overcome both of these limitations in children, the inventors established nasal-derived transcriptomic expression as a reliable surrogate of distal airway/bronchial expression. (Poole A, Urbanek C, Eng C, et al. Dissecting childhood asthma with nasal transcriptomics distinguishes subphenotypes of disease. The Journal of allergy and clinical immunology 2014;133:670-8.e12). The inventors found 90.2% of the 9,007 non-ubiquitously expressed bronchial genes were expressed in the nasal transcriptome, and the expression of these overlapping genes was strongly correlated between the nasal and bronchial airway (rho=0.87, Fig. 6). This is particularly relevant since there are similar patterns of elevation for the Th2 cytokines both at baseline and with virus in allergic airways. (Hansel TT, Tunstall T, Trujillo-Torralbo MB, et al. A Comprehensive Evaluation of Nasal and Bronchial Cytokines and Chemokines Following Experimental Rhinovirus Infection in Allergic Asthma: Increased Interferons (IFN-gamma and IFN-lambda) and Type 2 Inflammation (IL-5 and IL-13). EBioMedicine 2017;19:128-38).

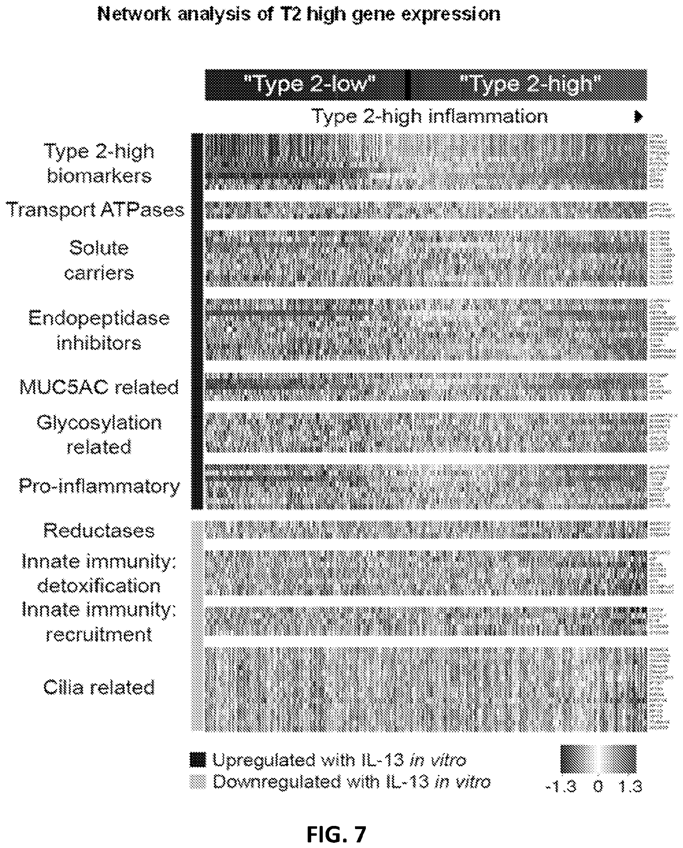

The inventors also identified a pattern of nasal Th2 inflammation enriched among asthmatics (O.R.-32.6), which mirrored the Th2 inflammation previously reported in asthmatic bronchial epithelium. Namely, asthmatics with nasal Th2 inflammation exhibited high nasal levels of IL13, IL4, IL5, periostin, CLCA1, and SERPINB2. (Poole A, Urbanek C, Eng C, et al. Dissecting childhood asthma with nasal transcriptomics distinguishes subphenotypes of disease. The Journal of allergy and clinical immunology 2014;133:670-8.e12). However, IL-4, IL-5 and IL-13 are expressed in low levels in bulk RNAseq due to lower frequency of mast cells and lymphocytes. Genes which are differentially expressed in single cell sequencing of nasal epithelial cells exposed to IL-13, which represent the majority of cells that would be obtained in epithelial swabs, were studied by others. These findings were validated in bulk RNAseq in nasal epithelial brushings from the GALA2 cohort (n=625). Specifically, co-expression-based gene network analysis identified a network of 47 genes which were highly enriched and well-established epithelial markers of T2 airway inflammation (e.g. *POSTN, CLCA1, IL1RL1, DPP4)* (Fig. 7). These genes were also highly correlated with both blood eosinophil level (Pearson's *r* = 0.56, p-value = 4.4 x 10⁻⁵⁹) and IgE (Pearson's r = 0.54, p-value = 1.3 x 10⁻⁴⁸). The same genes which were increased in IL-13 stimulated cells were increased in the epithelium of Th2 high individuals, validating this method to identify T2-high airway endotypes.

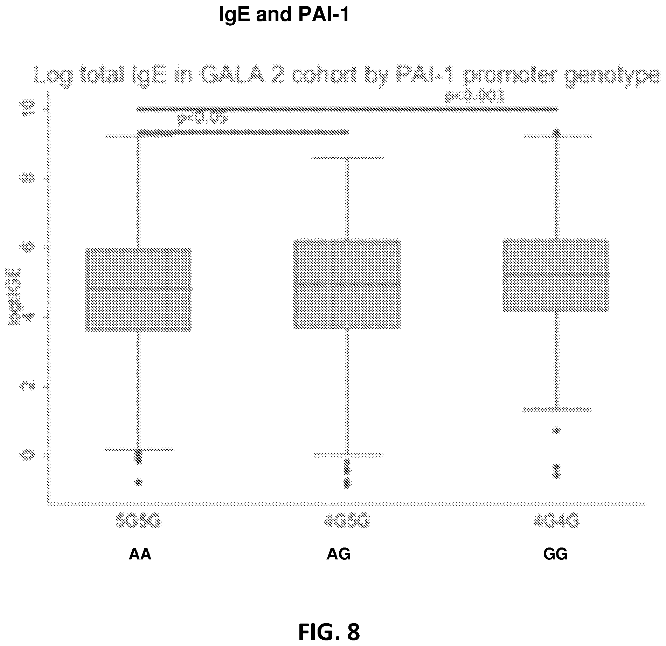

Finally, in the GALA2 cohort, the inventors have shown that a PAI-1 genotype in GWAS chips (rs2227631 A/G) which is in such close LD to the 4G/5G variant that it is used as a surrogate, is associated with serum IgE levels (Fig. 8). Others have also noted that PAI-1 is associated with increased local IgE production. (Liu RM, Eldridge S, Watanabe N, et al. Therapeutic potential of an orally effective small molecule inhibitor of plasminogen activator inhibitor for asthma. American journal of physiology Lung cellular and molecular physiology 2016;310:L328-36).

### Example 3 - Administration of soy isoflavones reduce allergic inflammation and improve airway hyperreactivity

The inventors have found that the administration of a therapeutically effective amount of soy isoflavones such as genistein, reduced allergic inflammation and improved airway hyperreactivity in a murine model of asthma.

### Experimental Plan

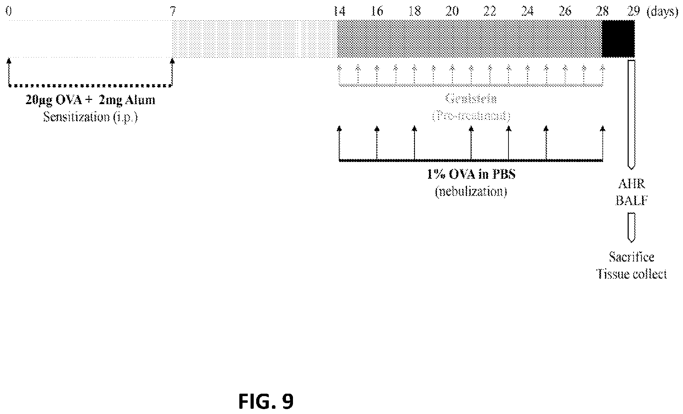

An overview of the experimental plan is shown in Figure 9. Eight-week-old BALB/c mice were purchased from The Jackson Lab and housed in standard laboratory conditions (12/12-h light/dark cycle, 22-24 °C, 40-60% humidity). Forty female mice were divided into four groups (namely, OVA+Veh, OVA+Bio300, PBS+Veh, PBS+Bio300). Genistein (Bio300, Humanetics Corp.) was administered to two of the groups in conjunction with either OVA or PBS. The mice in each group (*n =* 10) were equally divided into two cages (5 mice/cage) to provide appropriate space for the mice.

On Day 0 and Day 7, the groups for the first antigen presentation model (OVA+Veh, OVA+Bio300) received OVA (20ug OVA (grade:ll, Sigma-Aldrich, Saint Louis, MO, USA) emulsified in 2mg of Aluminum hydroxide in saline solution 100ul) by intra-peritoneal injection. The other antigen presentation model included PBS+Veh and PBS+Bio300 groups, which were given PBS.

From Day 14 to Day 28, the OVA+Bio300 and PBS+Bio300 groups received Bio300 (100ul) by oral gavage every other day. The other two groups (OVA+Veh and PBS+Veh) received control mixture. Moreover, the OVA+Veh and OVA+Bio300 groups received 20 ul of 1% OVA per 7 times via intranasal injection in 14, 16, 18, 20, 22, 24, 26, 28. The PBS groups received PBS intranasally. On Day 29, airway hyperresponsiveness (AHR) was assessed and tissues were obtained for further analysis.

### Airway Hyperreactivity

All mice were anesthetized with ketamine, tracheostomized, and mechanically ventilated with a FlexiVent system (SCIREQ, Flexivent, Scireq Inc., Montreal, Canada) as described in Choi et al. (Choi JM, Ahn MH, Chae WJ et al. Intranasal delivery of the cytoplasmic domain of CTLA-4 using a novel protein transduction domain prevents allergic inflammation. Nat Med 2006; 12:574-9). Volume changes due to thoracic expansion and alterations in tracheal pressure were measured in response to challenges with increasing concentrations of methacholine (3, 6, 12, 25, and 50 mg/mL) and reported as total respiratory system resistance (Rrs).

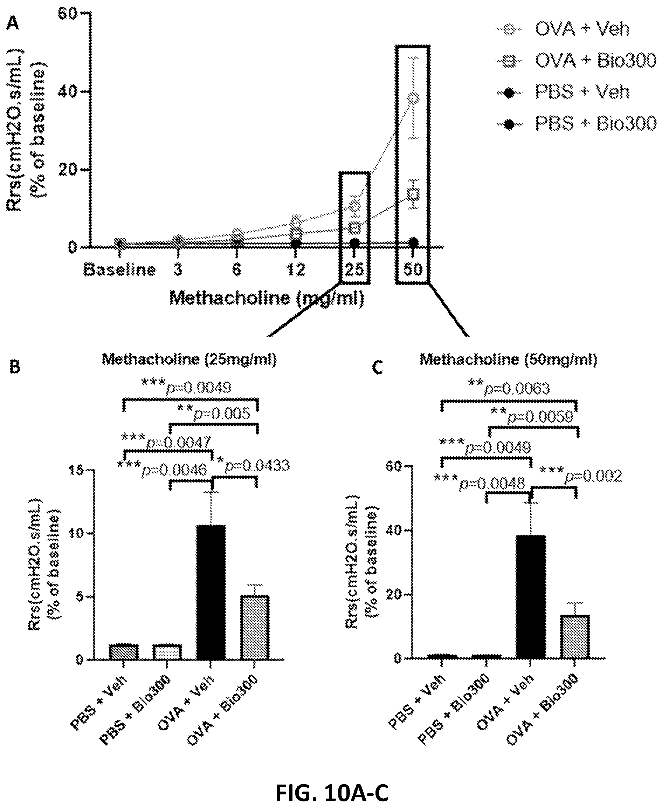

As shown in Figure 10A-C, the Rrs percentage was significantly decreased in the OVA+Bio300 group, followed by the OVA+Veh group (*p=0.0433) after administration of methacholine at a dose of 25 mg/ml. (Figure 10A and B). When the methacholine dose was doubled to 50 mg/ml, the Rrs percentage further decreased by about double for OVA+Bio300 followed by OVA+Veh (***p=0.002). (Figure 10A and C)

### Bronchoalveolar Lavage Fluid Analysis (BALF)

Mice were sedated and exsanguinated as described previously, and bronchoalveolar lavage (BAL) fluid was recovered after intratracheal instillation of 1 mL of PBS with lavage repeated twice (each time the amount collected was between 0.7-0.8 ml; total volume 1.5 ml).

Cell counts were performed by counting cells on cytospin slides. 0.2 mL of BAL was retrieved by cytocentrifugation, at 800 rpm for 5 min, onto slides. The cytospin slides were fixed and stained with Tek-Select^{®} Diff-Stain Kit (IMEB, Inc., San Marcos, CA, USA). Cytological examination for inflammatory cells were performed by brightfield microscope with x40 objective. The number of inflammatory cells counted from 10 random fields from each cytospin slides.

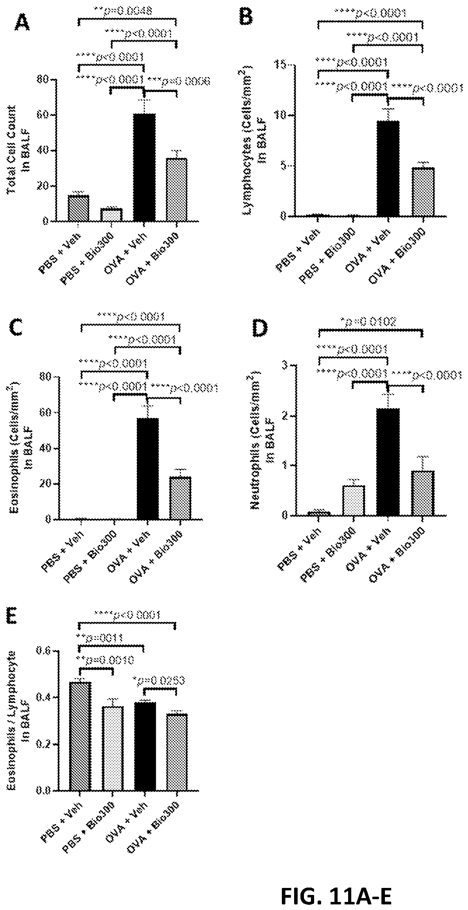

As shown in Figure 11A-D, the total cell count was significantly decreased in the OVA+Bio300 group versus the OVA+Veh group (***p=0.0006). (Figure 11A). The number of positive lymphocytes, eosinophils, and neutrophils were significantly decreased in the OVA+Bio300 group, followed by the OVA+Veh group (***p<0.0001). (Figure 11B-D). The eosinophils/lymphocytes ratio decreased in the OVA+Bio300 group, followed by the OVA+Veh group (*p=0.0253). (Figure 11E).

### Hematoxylin & Eosin (H&E), Periodic Acid-Schiff (PAS) and Trichome staining

All mice lungs were fixed with 10% neutral buffered formalin overnight. The tissues were then trimmed, processed and waxed according to the routine histological processes. Paraffin blocks were sectioned at 3 µm and stained with hematoxylin & eosin using an autostainer (Thermo Shandon, Pittsburgh, PA), periodic acid-Schiff (PAS) stain for identification of goblet cells in the epithelium, Masson's trichrome stain for airway fibrosis and cell counting was performed by counting at three random sites (x20) per slide under a light microscope.

As shown in Figure 12, the number of eosinophils and the eosinophils/lymphocytes ratio both were significantly decreased in the OVA+Bio300 group followed by the OVA+Veh group (****p<0.0001). A reduced patterns was observed in lymphocyte and neutrophil positive cells for the OVA+Bio300 group versus the OVA+Veh group. Further, total cell count was significantly decreased in the OVA+Bio300 group versus the OVA+Veh group (***p=0.0006).

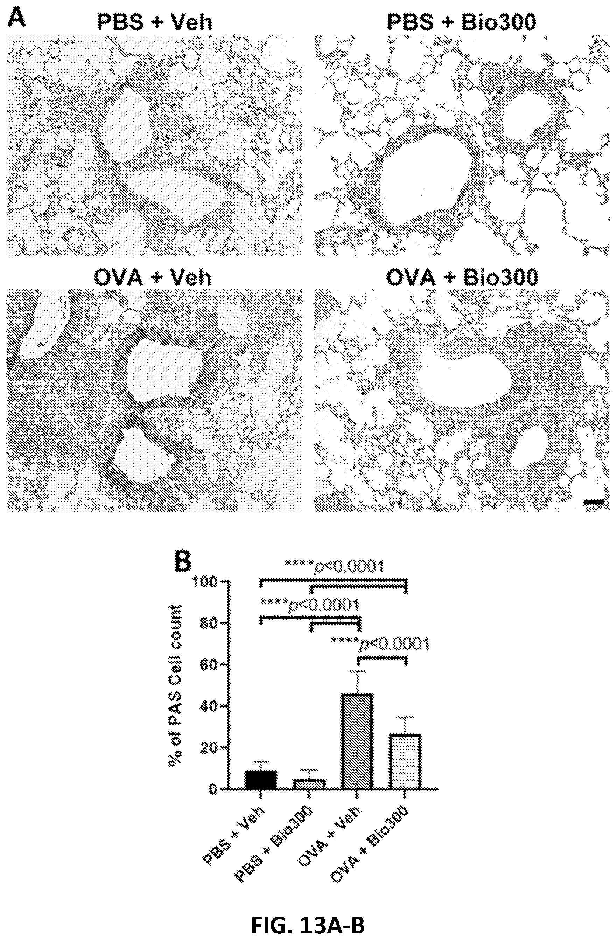

As shown in Figure 13A and B, the OVA+Veh group showed increased goblet cells as compared to the control group (****p<0.0001). Goblet cell numbers were also significantly decreased in the OVA+Bio300 group as compared to the OVA+Veh group (****p<0.0001).

### Conclusion

The inventors have found that administration of soy isoflavones in a murine model of asthma resulted in reduced allergic inflammation and improved airway hyperreactivity. These results provide support for the clinical trials described in the Examples below.

### Example 4 - Administration of soy isoflavones throughout the viral season to high risk infants with the PAI-1 genotype results in reduction in the proportion of subjects with a T2 airway endotype, decreased eosinophilic airway inflammation, and less aeroallergen sensitization (prophetic)

Based on the data obtained in the murine model described in Example 3, the inventors conduct trials in which a therapeutically effective dose of at least one soy isoflavone is administered to high risk infants having 4G4G or 4G5G PAI-1 risk genotype. The inventors determine that administration of a therapeutically effective amount of soy isoflavone results in fewer patients having a T2 airway endotype. The soy isoflavones decrease eosinophilic airway inflammation as supported with the results obtained in the murine studies of Example 3. Administration of soy isoflavones also results in less aeroallergen sensitization in patients.

### Trial Design

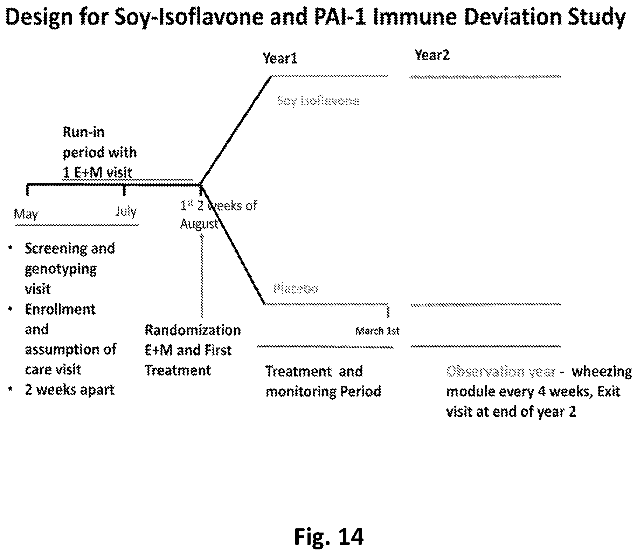

The inventors recruit and randomize high-risk infants aged 2-6 months and having the PAI-1 genotype (n=130) to a quadruple-masked, two arm, randomized placebo-controlled trial of supplementation with soy genistein (at doses similar to that achieved by soy formula ingestion) or matching placebo through the viral season (August 1^{st} to March 1^{st}) in 3 yearly cohorts. (Fig. 14)

### Eligibility

Infants who are at high risk of asthma (uni- or biparental asthma, or atopic dermatitis in the child) and who are 2-6 months of age at enrollment in the months of May - July prior to the peak HRV and RSV seasons are evaluated for enrollment. All subjects are genotyped and only those who are heterozygous or homozygous for the risk allele are randomized. Subjects are excluded if they are breastfeeding exclusively and do not include formula or expressed breast milk as at least 50% of the intake, if they will not modify existing formula or expressed breast milk by adding the study agent, or if they are already on a soy formula. Subjects are also excluded if already on a treatment for recurrent wheezing such as regular or intermittent inhaled steroids. In keeping with the prior SOYA study, infants are also excluded if their breastfeeding mothers are taking soy supplements or soy enriched foods more than 2 times a week and will not stop this level of ingestion. They are also excluded if they have congenital thyroid disease, a family history of estrogen sensitive mutations (e.g. BRACA1), or known hypersensitivity to the medications or soy protein. Subjects are also excluded for nonadherence including not taking soy/ placebo at least 80% of the time, not filling out diaries >80% in the enrollment period, and refusal of infant blood draw and nasal secretion measurement.

### Overview of Study Visits

Pre-randomization screening visits are conducted initially with in-person or phone contact to ensure basic eligibility in terms of age of child, inner city residence, and participant interest. Those interested are seen in person, consented, and have baseline questionnaires (demographics, general health, medication use, maternal diet including soy intake (Block Soy questionnaire), and wheezing history) administered for participant characterization and to determine eligibility. Subjects meeting inclusion undergo genotyping with those subjects having the genotype and meeting the inclusion criteria being enrolled with wheezing data, soy intake/dairy data, and medication review being performed.

A run-in period establishes a baseline over the months of May through July. There is a randomization visit at the end of this period (August) to ensure ongoing compliance with study protocol, and diary recording. Subjects are again assessed for unstable respiratory disease. Should subjects have more than one wheezing episode requiring prednisone during the run-in period, or a severe respiratory illness requiring ICU admission, they are removed from the trial.

Subjects are double-blind randomized (stratified by genotype) to either receive soy isoflavones or placebo. Baseline nasal swabs (2 copan flocked nasal swabs, one for T2 endotyping and one for an airway epithelial culture ) are taken. Baseline nasal secretions (by nasosorption to obtain Th2 cytokine levels, PAI-1 and ECP) are also taken. Phlebotomy is performed with specific and total IgE, PBMC and pDC isolation, plasma PAI-1, and plasma genistein being measured. Assessments are routinely taken every 4 weeks in both the treatment and the observation phases. Such assessments include any intercurrent exacerbations (systemic steroids or urgent same day visit, ED visit, or hospitalization), the ISAAC wheezing module, review of medication/ study drug dosing diaries, soy intake (non-study drug dietary intake) diaries, and any adverse events.

At the first sign of a viral illness, the subjects are brought in for assessment on day 3-5 of the illness (which corresponded with the highest airway T2 cytokine levels) to have nasal swabs and phlebotomy performed as well as an assessment of symptom scores and medication use. (Jackson DJ, Makrinioti H, Rana BM, et al. IL-33-dependent type 2 inflammation during rhinovirus-induced asthma exacerbations in vivo. Am J Respir Crit Care Med 2014;190:1373-82; Thwaites RS, Gunawardana NC, Broich V, et al. Biphasic activation of complement and fibrinolysis during the human nasal allergic response. The Journal of allergy and clinical immunology 2018;141:1892-5.e6; Thwaites RS, Ito K, Chingono JMS, et al. Nasosorption as a Minimally Invasive Sampling Procedure: Mucosal Viral Load and Inflammation in Primary RSV Bronchiolitis. The Journal of infectious diseases 2017;215:1240-4).

At the end of the treatment period, subjects are assessed via questionnaires with phlebotomy being performed to measure plasma PAI-1 levels, blood total and specific IgE, blood for PBMC and pDC studies. Nasal secretions and a nasal swab for T2 endotyping are performed. Subjects are given additional questionnaires and procedures related to adherence, adverse effects, and tolerability. Treatment adherence is assessed by diary review, weighing residual study drug and plasma levels of genistein, acknowledging the variability in genistein levels.

In the observation period (lasting one year after treatment) the inventors administer the same assessments every 4 weeks as per the visits earlier in the treatment period, and obtain intercurrent exacerbation history, the ISAAC wheezing module, review of any medications taken, and a soy intake questionnaire.

At the final end of study visit, plasma PAI-1 levels and blood for total IgE are obtained. Standardized study exit questionnaires and parent debriefing are provided at the final visit as well. Exit questionnaires determine global assessments of treatment, adequacy of informed consent procedures, satisfaction with study procedures and personnel, effectiveness of the masking procedures, and participation in future studies.

### Drug and Dosing

*Treatment Groups:* There are 2 parallel treatment groups: Soy isoflavone supplement (Novasoy) that contains soy genistein in levels comparable to that associated with soy formula intake (2.0 - 5.6 mg/kg), and matching placebo, both administered twice daily.

*Soy Isoflavone Dose:* The total amount of genistein in aglycone form in soy fed infants is estimated to range from 1.3-6.2 mg/kg body weight/day. To standardize doses dispensed, the inventors utilized the 50 percentile weight for 10 month old patients to calculate a 2.5 mg/kg dose for that weight (24.5 mg for males and 22.6 mg for females) for children aged 2-10 months. Similarly, the inventors utilized the 50 percentile weight for 24 month old patients to calculate a 2.5 mg/kg dose for that weight, given to children aged 10-24 months (31.8 mg for males and 30.3 mg for females). This results in a dose in the middle of the range of ingestion associated with formula, with extremes of a minimum of 2.0 mg/kg in in those at the 97 percentile at 10 and 24 months of age and a maximum of 5.6 mg/ kg in those at the 3rd percentile at 2 month of age (males and females). All doses are mixed with a palatable excipient powder to allow for a standardized dosing volume of 2.5 ml to for ease of dosing for families and facilitate placebo generation, which is the excipient only. Given the on demand nature of infant feeding, dosing twice daily (morning and evening) is administered.

*Treatment and Placebo Formulation:* The soy isoflavone is provided by Archer Daniels Midland in bulk and is compounded into the excipient powder to ensure consistent dosing by powder volume. The pharmacy facilitates a number of flavorings which can be added to excipients to maximize taste acceptability which are taste tested in volunteered age appropriate subjects to ensure palatability.

*Randomization and Treatment Distribution*/*medication handling:* Participants are randomly assigned to either active treatment or placebo on an equal allocation via permuted blocks with varying size, stratified by genotype. Neither participants nor the clinical center investigators are informed of the treatment group assignment to maintain the mask. The randomization sequence is generated by the study statistician and uploaded to the REDCap database, and randomization is implemented through REDCap, after all eligibility criteria have been checked, care assumed, compliance documented, and the participant's legal guardian has given written consent to enter the study. Only the treatment distribution center (study pharmacist) and the DCC know the actual treatment assignments. The study pharmacist provides an unmarked container of known weight containing either study drug or placebo, labeled with the patient's study ID, to the study coordinator conducting the randomization visit. At time of teaching around dosing, this container is provided to the family along with a standardized measure for 2.5 ml. The family brings the container for weighing at each in person visit.

*Biomarker status:* The inventors measure genistein levels as a measure of compliance. As expected, in the SOYA trial, genistein levels were significantly increased (103.2 ng/ml, p=0.0003) with soy isoflavone treatment in the group being studied (4G4G or 4G5G). Plasma levels of PAI-1, on the other hand, decreased with soy isoflavone treatment by a modest 5% in the 4G4G/4G5G group (-64 pg/ml p=0.051), and the effect on plasma PAI-1 levels are similar across genotypes (also noted in the 5G5G). These two findings suggest that blood levels may not be as relevant for exacerbations as airway levels (one of the key outcomes measured), which increase at times of viral illness and may be preferentially affected by genotype.

### Outcome Measures

### Primary Outcome: T2 endotype at the end of the treatment period

There are similar patterns of elevation in nasal T2 cytokines and bronchial T2 cytokines both at baseline and with virus making the nasal airway an attractive sampling site for pediatric studies where bronchoscopy is too invasive. However, use of nasal cytokines as a means of classification of subjects as T2 high or low is complicated by some overlap even between normal subjects and asthmatics for IL-5 and IL-13, and poor sensitivity of ELISA for IL-4 at the low levels noted in secretions. (Jackson DJ, Makrinioti H, Rana BM, et al. IL-33-dependent type 2 inflammation during rhinovirus-induced asthma exacerbations in vivo. Am J Respir Crit Care Med 2014;190:1373-82).

Alternately, T2 high elevation patterns in the nasal transcriptome have also been shown to mirror those in the lower airway. (Poole A, Urbanek C, Eng C, et al. Dissecting childhood asthma with nasal transcriptomics distinguishes subphenotypes of disease. The Journal of allergy and clinical immunology 2014;133:670-8.e12). The inventors previously identified a nasal T2 inflammation expression pattern (high levels of IL13, IL4, and IL5 as well as periostin, CLCA1, and SERPINB2) enriched among asthmatics (O.R.=32.6) that mirrored T2 inflammation previously reported in asthmatic bronchial epithelium. (Woodruff PG, Modrek B, Choy DF, et al. T-helper type 2-driven inflammation defines major subphenotypes of asthma. Am J Respir Crit Care Med 2009;180:388-95). Since then, as shown in Example 2 above, a validated T2 expression signature has been identified which is present on bulk RNA seq analysis of nasal epithelium. The inventors identified a subset of these including *POSTN, CLCA1, IL1RL1, DPP4, SERPINB2, ALOX15,* which includes markers previously identified and validated on bronchial biopsies as identifying T2 high asthma. (Woodruff PG, Modrek B, Choy DF, et al. T-helper type 2-driven inflammation defines major subphenotypes of asthma. Am J Respir Crit Care Med 2009;180:388-95). These markers are used to perform hierarchical clustering and designation of subjects as T2 high or low. Nasal Copan flocked swabs are used, which allow sufficient yield for determination of T2 endotype and viral load assessment at the viral visit (separate swabs).

### Planned Secondary outcomes

*Nasal* PAI-1, *ECP, and cytokine profile -* The inventors administer nasal absorption sampling at baseline, at day 3-5 of a viral illness, and at completion of treatment to determine levels of PAI-1, nasal ECP, and T2 cytokines/chemokines including TSLP, IL-4, IL-5, and IL13. IL-4, IL-5, and IL-13 responses are canonical cytokines that are pronounced in atopic asthmatics with Th2 airway inflammation at baseline and at times of viral illness. (Jackson DJ, Makrinioti H, Rana BM, et al. IL-33-dependent type 2 inflammation during rhinovirus-induced asthma exacerbations in vivo. Am J Respir Crit Care Med 2014;190:1373-82; Hansel TT, Tunstall T, Trujillo-Torralbo MB, et al. A Comprehensive Evaluation of Nasal and Bronchial Cytokines and Chemokines Following Experimental Rhinovirus Infection in Allergic Asthma: Increased Interferons (IFN-gamma and IFN-lambda) and Type 2 Inflammation (IL-5 and IL-13). EBioMedicine 2017;19:128-38; Thwaites RS, Gunawardana NC, Broich V, et al. Biphasic activation of complement and fibrinolysis during the human nasal allergic response. The Journal of allergy and clinical immunology 2018;141:1892-5.e6). Standardized filter paper swab (Hunt Developments^{™}) are placed in the nares and light pressure applied outside the nares for 60 seconds. Samples are placed on ice, processed within 3 hours and then stored at -80 degrees until assays are performed.

*Blood measures -* The inventors measure blood genistein (a measure of compliance/metabolism), periostin as an additional measure of T2 endotype), measures of sensitization specific IgEs for indoor allergens (Df, Dp, dog, cat, cockroach), as well as total IgE at baseline and 2 years of age. The specific IgE levels are a planned secondary analysis to evaluate effects of soy genistein on aeroallergen sensitization. These analyses include not only the level of specific IgE and change in specific IgE levels, but also evaluation of the number of allergens to which an infant becomes sensitized.

*Wheezing and respiratory morbidity -* As a prespecified secondary outcome, the inventors ascribe wheezing phenotypes and an asthma predictive index. The asthma predictive index is a validated tool used in longitudinal cohort studies and clinical trials of high-risk infants that has an 80% positive predictive value for persistent wheezing at age 6 years. (Guilbert TW, Morgan WJ, Zeiger RS, et al. Long-term inhaled corticosteroids in preschool children at high risk for asthma. N Engl J Med 2006;354:1985-97; Guilbert TW, Morgan WJ, Zeiger RS, et al. Atopic characteristics of children with recurrent wheezing at high risk for the development of childhood asthma. The Journal of allergy and clinical immunology 2004;114:1282-7; Zeiger RS, Mauger D, Bacharier LB, et al. Daily or intermittent budesonide in preschool children with recurrent wheezing. N Engl J Med 2011;365:1990-2001; Castro-Rodriguez JA, Holberg CJ, Wright AL, Martinez FD. A clinical index to define risk of asthma in young children with recurrent wheezing. Am J Respir Crit Care Med 2000;162:1403-6; Taussig LM, Wright AL, Holberg CJ, Halonen M, Morgan WJ, Martinez FD. Tucson Children's Respiratory Study: 1980 to present. The Journal of allergy and clinical immunology 2003;111:661-75; quiz 76; Castro-Rodriguez JA. The Asthma Predictive Index: a very useful tool for predicting asthma in young children. The Journal of allergy and clinical immunology 2010;126:212-6).

Children are classified as having a positive asthma predictive index at age 2-3 years. The wheezing phenotypes/trajectories are based on a standardized maternal questionnaire from the ISAAC wheezing module, the most widely accepted validated instrument used across multiple populations to estimate prevalence of asthma, administered every 4 weeks. (Worldwide variation in prevalence of symptoms of asthma, allergic rhinoconjunctivitis, and atopic eczema: ISAAC. The International Study of Asthma and Allergies in Childhood (ISAAC) Steering Committee. Lancet 1998;351:1225-32; Asher MI, Keil U, Anderson HR, et al. International Study of Asthma and Allergies in Childhood (ISAAC): rationale and methods. Eur Respir J 1995;8:483-91). Wheezing patterns assessed by this instrument have been shown to be prospectively associated with asthma morbidity and medication requirements in two birth cohorts. (Brunwasser SM, Gebretsadik T, Gold DR, et al. A new model of wheezing severity in young children using the validated ISAAC wheezing module: A latent variable approach with validation in independent cohorts. PloS one 2018;13:e0194739). The inventors determine number of episodes and also categorize children: no wheeze, occasional wheeze (1-2 episodes/last year), or recurrent wheeze (≥3 episodes/last year).

*Treatment adherence* - Treatment adherence is assessed by weighing remaining powder, questionnaire, and plasma genistein levels, acknowledging the variability in genistein levels. Soy use measures is not done in the presence of families to mitigate "dumping" behavior, allowing more accurate assessments.

*Side-Effects and Toxicity -* Side effects and toxicity are assessed by questionnaire and open-ended questions at each visit. Side-effects that are thought possibly or probably related to study treatment and are considered more than mild (i.e. interfering with daily functioning) will result in temporary discontinuation of study treatment. Serious adverse events (SAE) are reported immediately.

### Key Samples and Lab Measurements

PAI-1 *genotyping:* The genomic DNA is extracted with the QIAamp kit (Qiagen Inc, Valencia, CA), and the 4G5G genotyping is performed by an allele-specific PCR using an alternative forward primer, either GTCTGGACACGTGGGGG (SEQ ID NO: 1) for the 5G allele or GTCTGGACACGTGGGGA (SEQ ID NO: 2) for the 4G allele with a common reverse primer, TGCAGCCAGCCACGTGATTGTCTAG (SEQ ID NO: 3) and a control upstream primer, AAGCTTTTACC ATGGTAACCCCTGGT (SEQ ID NO: 4).

*PAI-1 level measurements:* Blood samples are collected either at 8:00 am or at a standard time of day for each visit, and stored in EDTA at -80º C. The concentration of PAI-1 in the blood of the subjects is measured in duplicate by ELISA (Assay Pro).

*Genistein level measurements:* Blood for total genistein concentration (unconjugated genistein plus genistein conjugated to glucuronide and sulfate) is collected in 10 ml heparinized Vacutainer tubes (BD, Franklin Lakes, New Jersey), centrifuged and the plasma collected and stored at -80ºC. A 200 µl-aliquot of plasma will be incubated in a solution containing β-glucuronidase and sulfatase overnight to de-conjugate genistein and extracted with diethyl ether. The mixture will be evaporated to dryness and the dry residues quantified by time-resolved fluoroimmunoassay (TR-FIA Genistein Kits, Labmaster, Aura, Finland).

*Nasal swabs for qt-PCR:* Nasal epithelial cells are collected from behind the inferior turbinate using a swab (COPAN flocked swabs, #107D13; Copan Diagnostics Inc). The collection swab is submerged in RLT Plus lysis buffer with β-mercaptoethanol and frozen at -80°C until extraction. Total RNA is extracted from all nasal swab samples, RNA quality control and quantitation performed (Agilent Bioanalyzer), and archived at -80°C. Quantitative real-time PCR for 6 genes, POSTN, CLCA1, IL1RL1, DPP4, SERPINB2, and ALOX15 is performed using methods described previously. (Kim DK, Jin HR, Eun KM, et al. The role of interleukin-33 in chronic rhinosinusitis. Thorax 2017;72:635-45). One microgram total RNA is reverse transcribed to cDNA using the cDNA Synthesis Kit (amfiRivert Platinum cDNA Synthesis MasterMix, GenDEPOT, Barker, Tx). For analysis of periostin (Hs01566734_m1), CLCA1 (Hs00976287_m1), IL1RL1 (Hs00249384 _m1), DPP4 (Hs00897386_m1), SERPINB2 (Hs01010736_m1), ALOX15 (Hs00993765_g1), and glyceraldehyde 3-phosphate dehydrogenase (GAPDH) (Hs02758991_g1), PDAR kits of primers and probes are purchased from TaqMan assays (ThermoFisher Scientific). Predeveloped assay reagent kits containing primers and probes are purchased from Applied Biosystems (Foster City, CA). Expression of GAPDH is used as an internal control for normalization. Cycling conditions are as follows: 95°C for 5 minutes followed by 60 cycles at 95°C for 15 seconds, 60°C for 20 seconds, and 72°C for 20 seconds. To analyze the data, Sequence Detection Software version 1.9.1 (Applied Biosystems) is used.

*Nasal secretions:* Nasal secretion collection by NasosorptionTM is performed prior to nasal swabs by placing standardized strips (4.5x27 mm) of a hydrophilic polyester synthetic absorptive matrix (SAM) (Mucosal Diagnostics, Hunt Developments (UK) Ltd) into each nostril for a duration of 60 seconds. The SAM is stored in -80°C for future elution. 100 µL of a PBS buffer containing 1% BSA and 1% Triton X (Sigma Aldrich, USA) is applied to the SAM prior to elution by spin filter centrifugation, and the eluate is immediately used for experiments or frozen at -80°C. Levels of PAI-1 (AssayPro, LLD 0.063 ng/ml, intra-assay CoV 5%, inter-assay CoV 9.7%), ECP (MyBiosource, LLD 0.1 ng/ml, intra-assay CoV <10%, inter-assay CoV <15%), IL-4 (R&D, LLD 10 pg/ml, intra-assay CoV 6.2%, inter-assay CoV 9.2), IL-5 (R&D, LLD 1.08 pg/ml, intra-assay CoV 4%, inter-assay CoV 7.9%), IL-13 (R&D, LLD 57.4 pg/ml, intra-assay CoV 2.4%, inter-assay CoV 5.9%), and TSLP (R&D, LLD 9.87 pg/ml, intra-assay CoV 8.2%, inter-assay CoV 7.5%) are analyzed using ELISA kits according to the manufacturer's protocols. The standard curve is derived using the recombinant protein provided per manufacturer's protocol.

*Sample handling plan:* All samples are labeled with study ID and transported to the research lab where they undergo initial processing. Blood samples are centrifuged and plasma is separated from the cells and stored in -80°C freezer with access control, 24 hour monitoring and backup power. The cells are used for isolation of PBMCs and pDCs. Other samples such as nasal secretions and nasal swabs are stored in -80°C for future experiments.

### Statistical Approach and Power

All analyses are carried out in an intent to treat fashion. Descriptive statistics are used to summarize baseline variables of interest overall and by arm. Residual diagnostics are examined and relevant assumptions are assessed using graphical display and statistical testing where appropriate. In situations where assumptions are in question, nonparametric methods or transformations may be applied. Every effort is made to minimize missing data; however, if warranted, multiple imputation methods may be considered and compared with estimates using complete cases.

The primary outcome, T2-high airway endotype, is treated as a binary endpoint. Primary analyses compare the proportion with T2-high airway endotype between arms at the end of the treatment period using a chi-squared test of independence. Secondary analyses compare all secondary continuous outcomes (e.g. cytokine levels, ECP, nasal and plasma PAI-1 and plasma genistein levels) between arms using two-sample t-tests or Wilcoxon rank sum tests, as appropriate. Additional secondary analyses compare secondary categorical variables (e.g. exacerbation status, wheezing status) between arms with chi-squared tests of independence. Incorporation of the randomization stratification factor (genotype) are considered in adjusted linear and logistic regression models, resulting in increased precision around the treatment effect of interest. Both unadjusted and adjusted analyses are presented, following CONSORT guidelines. A False Discovery Rate (FDR) correction is applied to all secondary analyses.

Subgroup analyses compare distributions of outcomes (e.g. T2 endotype, nasal cytokine levels, nasal ECP, nasal and plasma PAI-1 levels) between arms, within the subgroup experiencing a viral illness. Similar analyses are employed as described above. In the event of participants contributing data from multiple viral illnesses, a mixed model framework is employed to allow for inclusion of repeated measures.

Interim analyses for efficacy and safety are carried out via an O'Brien-Fleming type alpha-spending function with the following equation: α (t) = 2 - 2 phi (Z α/2 /√t ). This analysis is conducted after the follow-up data have been collected from the second cohort. Interim analyses are carried out to determine if there is strong evidence of efficacy (significantly reduced proportion of T2-high airway endotype) or safety concerns (significantly increased proportion of subjects with exacerbations) that would warrant stopping the trial early.

*Power:* Power considerations were based on determining the necessary sample size to detect a 50% relative reduction in proportion with T2-high airway endotype in the treatment arm compared to the control. Approximately ½ of asthmatics have airway eosinophilia and a signature in airway epithelial cells which is associated with IL-13. Thus, assuming a proportion with T2-high airway endotype in the control arm of 0.50, a total sample size of 116 participants would provide 80% power to detect an absolute difference in proportions of 0.25 between arms. Power calculations assumed a two-sided type I error rate of 0.05. In order to account for 10% loss to follow-up by the end of the treatment period, the necessary sample size was inflated to 130 participants to ensure adequate power.

### Rigor and reproducibility

*Data Management Plan:* A dedicated REDCap database is designed, built and maintained by the study team. REDCap is securely housed at Northwestern University's Clinical and Translational Sciences Institute (NUCATS). All data access is audited, and role-based security is used to provide seamless access and integration to functions such as data entry, data download, and the generation of reports. To ensure the integrity and reliability of data collected, all the eCRFs are first built in the REDCap database. The eCRFs in REDCap mirror the individual item assessments/fields in the paper CRFs to minimize discrepancies with interpretation or human error upon data entry. Each field in REDCap contains a unique variable name to differentiate it from others. Field validations and required entry flags are programmed into the REDCap project. Once CRFs and eCRFs are completely drafted, a series of mock trials are conducted.

*Data and Safety Monitoring Plan:* The data and safety monitoring approach relies on Data Status and Quality Reports (DSQRs) and REDCap reporting features. The inventors use the REDCap Application Programming Interface (API) functionality to export the study data, and then restructure and summarize the data using statistical software such as R or SAS. The reports are reviewed and discussed weekly, on average, but once the systems are in place and code generated, then the reports may be updated in real-time. Data checks are also performed to confirm internal consistency of data. Missing fields are listed. If errors or missing values are identified, the clinical team resolves all issues. The REDCap Data Resolution Workflow is used to monitor resolution of all data queries. DSQRs may continually evolve throughout the course of the study, but they focus on essential, aggregated study data including: baseline information (screening and consent, enrollment, demographics), AEs/SAEs, and other pre-specified variables of interest.

*Sex* as a *Biological Variable:* The inventors have evaluated the differences in the effect of soy isoflavones in individuals with the PAI-1 genotype by sex in the SOYA study and found no sex differences. However, upregulation of estrogen receptors may be a compensatory response to allergen induced airway responses. To evaluate the effect of sex, data is analyzed to determine if there are differences by sex on airway cytokines, ECP, and proportion of T2 high subjects. If there are differences by sex, the effects of soy isoflavone are evaluated to determine any difference by sex hormones, assaying the hormone levels at baseline and treatment completion.

### Alternatives

*Effect of inhaled steroids on outcomes* - Children are only enrolled if they are not already on a medication for recurrent wheezing, however subsequent administration of an inhaled steroid is not controlled. The primary focus is progression to a T2 airway endotype, and inhaled steroids have not been shown to decrease likelihood of progression to asthma. The inventors determine if there are differences in the groups by use of inhaled steroid and if not, the data is pooled. If there seem to be differences and there are a large number of children on inhaled steroid, secondary analyses are carried out and stratified by inhaled steroid use to describe differences. Alternately, if as in the URECA study, there are only 14% with a modified asthma predictive index (and therefore only a smaller subset of these on inhaled steroid), the inventors carry out sensitivity analyses removing children who are on inhaled steroid.

*Unexpected outcome* - If a difference in T2 endotypes between groups is not shown despite clinical improvement, the inventors carry out full transcriptomics of nasal brushings both at the times of viral illness, and also at the completion of therapy to agnostically examine the pathways associated with soy isoflavone administration.

*Swabs vs. Brushings -* Finally, the inventors are carrying out nasal swabs and not brushings. While brushings result in 9.8x10⁵ cells, swabs result in similar number of cells 2.4x10⁵ cells with similar viability, but slightly lower primary cell culture success (65%) compared to brushings (90%). However, cell culture is not part of the current protocol. Swabs are more acceptable, and have been carried out in large population based cohorts such as the CHILD study which collected 3542 swabs in 3 year old children for the purposes of both RNAseq and cell culture. (Subbarao P, Anand SS, Becker AB, et al. The Canadian Healthy Infant Longitudinal Development (CHILD) Study: examining developmental origins of allergy and asthma. Thorax 2015;70:998-1000). Even if it is later decided to perform cell culture in ancillary studies, each child is swabbed at 2 time points outside of viral illness, decreasing the culture fail rate to 12%, and yielding adequate swabs for any ancillary studies.

### Example 5 - Administration of soy isoflavones improve rhinovirus-induced IFN-α responses in PBMC's by affecting the PAI-1-IgE axis in subjects with the PAI-1 risk genotype (prophetic)

Soy isoflavone treatment has been shown to reduce severe asthma exacerbation by four fold in asthma patients with the high PAI-1 genotype compared to placebo but not in those with the low PAI-1 producing genotype. The inventors also showed that genistein, a major isoflavone in soy, significantly inhibited TGF-β1-induced PAI-1 production from human airway EpC at least in part via the AMPK pathway. (Cho SH, Jo A, Casale T, et al. Soy isoflavones reduce asthma exacerbation in asthmatic patients with high PAI-1-producing genotypes. The Journal of allergy and clinical immunology 2019). It is not clear how decreased PAI-1 production by soy isoflavones reduces severe asthma exacerbations. However, the data show that PAI-1 enhances TLR-3 agonist/IL-4-induced TSLP production from airway EpC18, which suggests that soy isoflavones reduce TSLP production via decreasing PAI-1 induction of TSLP in viral asthma exacerbations. Interestingly, total blood IgE levels are significantly higher in the high PAI-1 producing genotype, 4G4G, compared to the 5G5G genotype, suggesting a connection between PAI-1 and IgE levels through the PAI-1-TSLP axis. Blocking IgE by omalizumab decreased the duration of human rhinovirus (HRV) infections, viral shedding, and risk of HRV-related illness compared with guideline-driven care alone. (Esquivel A, Busse WW, Calatroni A, et al. Effects of Omalizumab on Rhinovirus Infections, Illnesses, and Exacerbations of Asthma. Am J Respir Crit Care Med 2017;196:985-92). Blocking IgE also attenuates pDC FcεRla expression, augmenting pDC IFN-α responses to HRV. (Gill MA, Liu AH, Calatroni A, et al. Enhanced plasmacytoid dendritic cell antiviral responses after omalizumab. The Journal of allergy and clinical immunology 2018;141:1735-43.e9). These findings suggest that reduced blood IgE levels and pDC FcεRlα expression by soy isoflavones may decrease susceptibility to respiratory viral illnesses through enhanced IFN-α responses in pDCs, a mechanism by which soy isoflavone-treated asthmatics may have improved anti-viral IFN-α responses.

The inventors isolate peripheral blood mononucleated cells (PBMCs) and pDCs from the subjects in Example 4 (soy isoflavones and placebo group, n=58 each group). The inventors measure IFN-α levels in the supernatants from PBMCs as the primary outcome after stimulation with IgE receptor cross-linking plus HRV, and compare samples from control subjects to those treated with soy isoflavones. Secondarily IFN-α levels are measured in the supernatants from pDcs and FcεRlα expression on pDCs and PBMCs. The inventors also measure IFN-α and viral load based on nasal secretions/nasal swab on the samples obtained in Example 4 at the time of a viral illness.

### Experimental design

### Isolation and culture of PBMCs and pDCs

To test that soy isoflavones improve rhinovirus-induced IFN-α responses in PBMCs and pDCs by affecting the PAI-1-IgE axis in subjects with the PAI-1 risk genotype, PBMCs are isolated by Ficoll-Paque and pDCs are purified from PBMCs by means of negative selection with antibody-coated magnetic particles STEMCELL Technologies, Vancouver, Canada). (Gill MA, Liu AH, Calatroni A, et al. Enhanced plasmacytoid dendritic cell antiviral responses after omalizumab. The Journal of allergy and clinical immunology 2018;141:1735-43.e9). EasySep Purity of the isolated pDCs (defined as Lin-HLA-DR+CD11c-CD123+ events) is greater than 80%. pDCs are distinguished from basophils by means of HLA-DR expression. Aliquots of PMBCs and pDCs are preserved in Cyto-Chex solution (Streck, Omaha, NE) for subsequent antibody staining and flow cytometric analysis. Cells are cultured at 0.5 × 10⁶/0.2 mL (PBMCs) or 0.1 × 10⁵/0.2 mL (pDCs) in 96-well round-bottom plates in complete RPMI 1640 medium supplemented with 10% heat-inactivated FBS, 1% penicillin-streptomycin, 1% sodium pyruvate, 1% HEPES buffer solution, 1% nonessential amino acids, 1% glutamate, 100 µmol/L β-mercaptoethanol, and 10 ng/mL IL-3. From 1 cc of blood, 0.2-0.6 × 10⁵ pDCs are isolated. Therefore, 4-5ccs of blood is sufficient to conduct the experiments.

Cells are cultured for 18 hours in the absence or presence of an IgE cross-linking antibody (rabbit anti-human IgE, 0.1, 1, or 10 µg/mL; Bethyl Laboratories, Montgomery, TX). This antibody has the ability to bind and cross-link IgE in cell-surface FcεRl receptors. After 18 hours, cells are stimulated with RV-A16 (at multiplicity of infection (MOI) of 0.1, 1 or 5; a gift from Dr. Mike Teng's virology lab, University of South Florida) or gardiquimod (a TLR7 agonist, 1 µg/mL; InvivoGen, San Diego, CA) for 24 hours. Cell supernatants are then harvested by means of centrifugation and stored at -80°C for IFN-α ELISA.

### Flow cytometry

To confirm that soy isoflavones reduce circulating and tissue levels of IgE and further FcεRI expression on pDCs, pDCs are isolated as described above. Then FcεRI expression are measured using FcεRIα-PE (eBioscience, San Diego, CA); acquired on a BD LSR II flow cytometer (BD Biosciences); and analyzed by using FlowJo software TreeStar, Ashland, OR). (Gill MA, Liu AH, Calatroni A, et al. Enhanced plasmacytoid dendritic cell antiviral responses after omalizumab. The Journal of allergy and clinical immunology 2018;141:1735-43.e9). The mean fluorescence intensity of FcεRIα is determined by using flow cytometry and subsequently converted to molecules of equivalent soluble fluorochrome by using the Ultra Rainbow Calibration Kit (BD Biosciences). Post-/pre-treatment ratio of mean equivalent soluble fluorochrome values in the soy isoflavone group and placebo group is analyzed (mean ± 95% CI).

### IFN-α quantification

To confirm that soy isoflavones improve rhinovirus-induced IFN-α responses in PBMCs and pDCs in subjects with the PAI-1 risk genotype, IFN-α protein levels in nasal secretions from subjects in Example 4 and cell supernatants in in this Example are measured by using the Human IFN-α ELISA Kit as described in Gill et al.

(Gill MA, Liu AH, Calatroni A, et al. Enhanced plasmacytoid dendritic cell antiviral responses after omalizumab. The Journal of allergy and clinical immunology 2018;141:1735-43.e9). The lower limit of detection is 4 pg/ml. In the cell experiments, the inventors measure pre-/post-randomization means and compare the post/pre-treatment ratio of geometric means in soy isoflavone treatment group and placebo group.

### Rhinovirus viral load

To confirm that soy isoflavones reduce RV viral load in the airways by increased rhinovirus-induced IFN-α responses in subjects with the PAI-1 risk genotype, RNA is extracted from nasal swab samples during viral illness (QIAamp viral RNA mini kit; Qiagen) and reverse-transcribed (omniscript RT kit, Qiagen) with random hexamers. Standard RV PCR (PerkinElmer 9600 GeneAmp) is performed from 2 µL of cDNA as described in Message et al. (Message SD, Laza-Stanca V, Mallia P, et al. Rhinovirus-induced lower respiratory illness is increased in asthma and related to virus load and Th1/2 cytokine and IL-10 production. Proc Natl Acad Sci U S A 2008;105:13562-7). The inventors measure viral load during viral illness in the soy isoflavones and placebo groups compare the means (mean ± SE).

### Statistical Design and Sample Size

As in Example 4, analyses are carried out in an intent to treat fashion; similar descriptive statistics and assessment of assumptions and missing data are employed as described above. The primary outcome, IFN-α from PBMCs, is treated as a continuous variable and log-transformed for analyses. Primary analyses compare the log-transformed distributions of INF-α, after stimulation with IgE receptor cross-linking plus HRV, between arms using independent two-sample t-tests. Secondary analyses compare distributions of IFN-α from pDCs and FcεRI-α expression on pDCs using similar approaches, and incorporating log transformations for skewed outcomes, as appropriate. Additional secondary analyses consider the subgroup of participants presenting with a viral illness. IFN-α and viral load distributions at the time of viral illness are compared between arms within this subgroup using two-sample t-tests. A mixed model framework may be employed, as described in Example 4, should participants contribute data from multiple viral illnesses. Incorporation of the randomization stratification factor (genotype) is considered in adjusted linear regression models, resulting in increased precision around the treatment effect of interest. Both unadjusted and adjusted analyses are presented, following CONSORT guidelines. A False Discovery Rate (FDR) correction is applied to all secondary analyses.

### Power

Power considerations were based on detecting a difference in mean log-transformed INF-α on PBMC between arms of at least 1.17, translating to a ratio of geometric means of 3.22. This effect size is a similar order of magnitude to what was seen with omalizumab, and hence what is expected to be clinically relevant. Assuming standard deviations on the log scale of 1.86 in the control arm and 2.10 in the treated arm, a sample size of 47 per arm would be required to detect this difference with 80% power. Thus, the proposed sample size of 116 based on Example 4 analyses provides at least 80% power to detect differences as small as 1.04, translating to a ratio of geometric means of 2.83. Power calculations assumed a two-sample t-test with type I error rate of 0.05, and as described above, the total sample size was inflated to 130 participants to account for 10% loss to follow-up.

### Results

The inventors expect that anti-lgE/RV-treated IFN-α levels in the supernatants from PBMCs and pDCs increase in the post-soy isoflavone treatment group compared to baseline levels (pre-randomization). On the other hand, there is no difference in the levels of IFN-α between post-treatment and pre-randomization in the placebo group, implying that soy isoflavones improve the anti-viral IFN-α response to restore innate immune function and this may contribute to reduced RV viral load. However, it is possible that IFN-α levels are not significantly increased in either PBMCs or pDCs after soy isoflavone treatment. If so, the inventors examine whether there is any significant difference in levels between the 4G4G and 4G5G genotype groups because there may be a difference in PAI-1 production between the two genotypes. If IFN-α levels are not significantly increased in either PBMCs or pDCs or both cell types after soy isoflavone treatment, but increased in nasal secretions during viral illness in the soy isoflavone treated group compared to placebo, soy isoflavone treatment may influence type I IFN response from other airway cell types such as airway macrophages.

The inventors expect that the expression of FcεRIα on pDCs is significantly decreased in the post-soy isoflavone treatment group compared to the baseline (pre-randomization). In a previous study, the expression of FcεRIα on pDCs was reduced about 57% by omalizumab treatment. The inventors expect that soy isoflavone treatment is less effective, but at least 25-35% reduction of the FcεRIα expression on pDCs. However, the inventors expect no difference in FcεRIα expression between post-treatment and pre-randomization in the placebo group. This finding suggests that soy isoflavones reduce IgE production and FcεRIα expression on pDCs. The inventors also expect that rhinovirus viral load is decreased in the soy isoflavone group in keeping with increased IFN-α production. If there is no significant decrease in FcεRIα expression or significant decrease in RV viral load in the soy isoflavone group, the inventors examine whether there is any significant difference between the 4G4G and 4G5G genotype groups as explained above. If there is no significant decrease in FcεRIα expression on pDCs with soy isoflavone treatment but IFN-α levels are increased in nasal secretions and viral load during illness is decreased, this may again suggest that soy isoflavone treatment may influence type I IFN response via other cell types in the airways. The inventors also correlate the type I IFN response with clinical outcomes to see if modulation of type I IFN responses are associated with the clinical effects of soy isoflavones.

### Example 6 - Prophylactic treatment with soy isoflavones in a pediatric patient (prophetic)

A 12 month old female child is at high risk for asthma development due to having both parents suffer from asthma as well as exhibiting atopic dermatitis.

The child also suffered from a respiratory virus (RSV) infection at 8 months of age which required medical care. The asthma predictive index is administered and positively identifies a high risk of asthma. Nasal swabs are taken from the child and nasal-derived transcriptomic expression is performed to determine PAI-1 genotype of the child. The child is found to have a 4G4G PAI-1 risk genotype, which is a risk factor for development of asthma. The child is administered a therapeutically effective amount of genistein daily in an amount of 30.3 mg per day. The child does not develop asthma.

### Conclusion

The inventors find that administration of a therapeutically effective amount of at least one soy isoflavone is capable of preventing the development of asthma in pediatric patients having the PAI-1 risk genotype of 4G4G or 4G5G. The inventors also find soy isoflavone administration reduces respiratory virus viral load in the airways by increased rhinovirus-induced IFN-α responses. Children with the risk genotype (homo or heterozygous) with a severe viral illness have less PAI-1 in the airway, greater IFN-α production during viral illness, and ultimately have decreased risk of developing Th2 airway endotypes if treated with soy isoflavones during viral seasons.

It is also to be understood that the following claims are intended to cover all of the generic and specific features of the invention herein described, and all statements of the scope of the invention which, as a matter of language, might be said to fall there between. Now that the invention has been described,

## Claims

1. A soy isoflavone for use in a method of preventing asthma development in a patient having a plasminogen activator inhibitor 1 (PAI-1) risk genotype, wherein:
a PAI-1 genotype of the patient is determined; and
a therapeutically effective amount of at least one soy isoflavone is administered to the patient if the PAI-1 risk genotype is 4G4G or 4G5G wherein the at least one soy isoflavone is administered in the absence of any additional therapeutic agents;
wherein the administration of the therapeutically effective amount of the at least one soy isoflavone to the patient prevents the asthma development.

2. The soy isoflavone for use according to claim 1, wherein the at least one soy isoflavone is selected from the group consisting of genistein, daidzein, and glycitein.

3. The soy isoflavone for use according to claim 1, wherein the at least one soy isoflavone is administered to the patient orally.

4. The soy isoflavone for use according to claim 1, wherein the at least one soy isoflavone is administered daily.

5. The soy isoflavone for use according to claim 4, wherein the therapeutically effective amount of the at least one soy isoflavone is administered to the patient during a viral season.

6. A soy isoflavone for use in a method of preventing asthma development in a pediatric patient having a plasminogen activator inhibitor 1 (PAI-1) risk genotype, wherein:
a PAI-1 risk genotype of the pediatric patient is determined; and
a therapeutically effective amount of at least one soy isoflavone is administered to the patient if the PAI-1 risk genotype of the patient is 4G4G or 4G5G wherein the at least one soy isoflavone is administered in the absence of any additional therapeutic agents;
wherein the administration of the therapeutically effective amount of the at least one soy isoflavone to the pediatric patient prevents the asthma development.

7. The soy isoflavone for use according to claim 6, wherein the pediatric patient is at high risk of the asthma development due to uni- or biparental asthma or atopic dermatitis.

8. The soy isoflavone for use according to claim 6, wherein the at least one soy isoflavone is selected from the group consisting of genistein, daidzein, and glycitein.

9. The soy isoflavone for use according to claim 6, wherein the at least one soy isoflavone is administered to the patient orally.

10. The soy isoflavone for use according to claim 6, wherein the therapeutically effective amount of the at least one soy isoflavone is administered to the pediatric patient during a viral season.

11. The soy isoflavone for use according to claim 6, wherein the at least one soy isoflavone is administered daily.

12. A soy isoflavone for use in a method of determining responsiveness to soy isoflavone administration for prevention of asthma development in a patient in need thereof, wherein:
a plasminogen activator inhibitor 1 (PAI-1) risk genotype of the patient is determined; and
a therapeutically effective amount of at least one soy isoflavone is administered to the patient prior to the development of the asthma if the PAI-1 risk genotype is 4G4G or 4G5G wherein the at least one soy isoflavone is administered in the absence of any additional therapeutic agents ;
wherein the PAI-1 risk genotype of 4G4G or 4G5G is responsive to the prevention of the asthma in the patient.

13. The soy isoflavone for use according to claim 12, wherein the at least one soy isoflavone is administered to the patient orally.

14. The soy isoflavone for use according to claim 12, wherein the at least one soy isoflavone is selected from the group consisting of genistein, daidzein, and glycitein.

## Patentansprüche

1. - Soja-Isoflavon zur Verwendung in einem Verfahren zum Verhindern einer Entwicklung von Asthma bei einem Patienten, der einen Risikogenotyp Plasminogen-Aktivator-Hemmer 1 (PAl-1) hat, wobei:
ein PAl-1-Genotyp des Patienten bestimmt wird; und
eine therapeutisch wirksame Menge mindestens eines Soja-Isoflavons an den Patienten verabreicht wird, wenn der PAl-1-Risikogenotyp 4G4G oder 4G5G ist, wobei das mindestens eine Soja-Isoflavon in Abwesenheit beliebiger zusätzlicher therapeutischer Mittel verabreicht wird;
wobei die Verabreichung der therapeutisch wirksamen Menge des mindestens einen Soja-Isoflavons an den Patienten die Entwicklung von Asthma verhindert.

2. - Soja-Isoflavon zur Verwendung nach Anspruch 1, wobei das mindestens eine Soja-Isoflavon ausgewählt ist aus der Gruppe, bestehend aus Genistein, Daidzein und Glycitein.

3. - Soja-Isoflavon zur Verwendung nach Anspruch 1, wobei das mindestens eine Soja-Isoflavon dem Patienten oral verabreicht wird.

4. - Soja-Isoflavon zur Verwendung nach Anspruch 1, wobei das mindestens eine Soja-Isoflavon täglich verabreicht wird.

5. - Soja-Isoflavon zur Verwendung nach Anspruch 4, wobei die therapeutisch wirksame Menge des mindestens einen Soja-Isoflavons dem Patienten während einer Virussaison verabreicht wird.

6. - Soja-Isoflavon zur Verwendung in einem Verfahren zum Verhindern einer Entwicklung von Asthma bei einem pädiatrischen Patienten, der einen Risikogenotyp Plasminogen-Aktivator-Hemmer 1 (PAl-1) hat, wobei:
ein PAl-1-Risikogenotyp des pädiatrischen Patienten bestimmt wird; und
eine therapeutisch wirksame Menge mindestens eines Soja-Isoflavons an den Patienten verabreicht wird, wenn der PAl-1-Risikogenotyp des Patienten 4G4G oder 4G5G ist, wobei das mindestens eine Soja-Isoflavon in Abwesenheit beliebiger zusätzlicher therapeutischer Mittel verabreicht wird;
wobei die Verabreichung der therapeutisch wirksamen Menge des mindestens einen Soja-Isoflavons an den pädiatrischen Patienten die Entwicklung von Asthma verhindert.

7. - Soja-Isoflavon zur Verwendung nach Anspruch 6, wobei der pädiatrische Patient ein hohes Risiko für die Entwicklung von Asthma aufgrund von uni- oder biparentalem Asthma oder atopischer Dermatitis hat.

8. - Soja-Isoflavon zur Verwendung nach Anspruch 6, wobei das mindestens eine Soja-Isoflavon ausgewählt ist aus der Gruppe, bestehend aus Genistein, Daidzein und Glycitein.

9. - Soja-Isoflavon zur Verwendung nach Anspruch 6, wobei das mindestens eine Soja-Isoflavon dem Patienten oral verabreicht wird.

10. - Soja-Isoflavon zur Verwendung nach Anspruch 6, wobei die therapeutisch wirksame Menge des mindestens einen Soja-Isoflavons dem pädiatrischen Patienten während einer Virussaison verabreicht wird.

11. - Soja-Isoflavon zur Verwendung nach Anspruch 6, wobei das mindestens eine Soja-Isoflavon täglich verabreicht wird.

12. - Soja-Isoflavon zur Verwendung in einem Verfahren zum Bestimmen eines Ansprechens auf eine Verabreichung von Soja-Isoflavon zum Verhindern einer Entwicklung von Asthma bei einem Patienten, der dies benötigt, wobei:
ein Risikogenotyp Plasminogen-Aktivator-Hemmer 1 (PAl-1) des Patienten bestimmt wird; und
eine therapeutisch wirksame Menge mindestens eines Soja-Isoflavons an den Patienten vor der Entwicklung des Asthmas verabreicht wird, wenn der PAl-1-Risikogenotyp 4G4G oder 4G5G ist, wobei das mindestens eine Soja-Isoflavon in Abwesenheit beliebiger zusätzlicher therapeutischer Mittel verabreicht wird;
wobei der PAl-1-Risikogenotyp 4G4G oder 4G5G auf die Vorbeugung von Asthma bei dem Patienten anspricht.

13. - Soja-Isoflavon zur Verwendung nach Anspruch 12, wobei das mindestens eine Soja-Isoflavon dem Patienten oral verabreicht wird.

14. - Soja-Isoflavon zur Verwendung nach Anspruch 12, wobei das mindestens eine Soja-Isoflavon ausgewählt ist aus der Gruppe, bestehend aus Genistein, Daidzein und Glycitein.

## Revendications

1. - Isoflavone de soja pour utilisation dans une méthode de prévention du développement de l'asthme chez un patient ayant un génotype de risque d'inhibiteur de l'activateur du plasminogène 1 (PAI-1), dans laquelle :
le génotype PAI-1 du patient est déterminé ; et
une quantité thérapeutiquement efficace d'au moins une isoflavone de soja est administrée au patient si le génotype de risque PAI-1 est 4G4G ou 4G5G, l'au moins une isoflavone de soja étant administrée en l'absence de n'importe quels autres agents thérapeutiques ;
l'administration de la quantité thérapeutiquement efficace de l'au moins une isoflavone de soja au patient prévenant le développement de l'asthme.

2. - Isoflavone de soja pour utilisation selon la revendication 1, dans laquelle l'au moins une isoflavone de soja est choisie dans le groupe consistant en la génistéine, la daidzéine et la glycitéine.

3. - Isoflavone de soja pour utilisation selon la revendication 1, dans laquelle l'au moins une isoflavone de soja est administrée au patient par voie orale.

4. - Isoflavone de soja pour utilisation selon la revendication 1, dans laquelle l'au moins une isoflavone de soja est administrée quotidiennement.

5. - Isoflavone de soja pour utilisation selon la revendication 4, dans laquelle la quantité thérapeutiquement efficace de l'au moins une isoflavone de soja est administrée au patient pendant une saison virale.

6. - Isoflavone de soja pour utilisation dans une méthode de prévention du développement de l'asthme chez un patient pédiatrique ayant un génotype de risque d'inhibiteur de l'activateur du plasminogène 1 (PAI-1), dans laquelle :
un génotype de risque PAI-1 du patient pédiatrique est déterminé ; et
une quantité thérapeutiquement efficace de l'au moins une isoflavone de soja est administrée au patient si le génotype de risque PAI-1 du patient est 4G4G ou 4G5G, l'au moins une isoflavone de soja étant administrée en l'absence de n'importe quels autres agents thérapeutiques ;
l'administration de la quantité thérapeutiquement efficace de l'au moins une isoflavone de soja au patient pédiatrique prévenant le développement de l'asthme.

7. - Isoflavone de soja pour utilisation selon la revendication 6, dans laquelle le patient pédiatrique présente un risque élevé de développement de l'asthme en raison d'un asthme uni- ou biparental ou d'une dermatite atopique.

8. - Isoflavone de soja pour utilisation selon la revendication 6, dans laquelle l'au moins une isoflavone de soja est choisie dans le groupe consistant en la génistéine, la daidzéine et la glycitéine.

9. - Isoflavone de soja pour utilisation selon la revendication 6, dans laquelle l'au moins une isoflavone de soja est administrée au patient par voie orale.

10. - Isoflavone de soja pour utilisation selon la revendication 6, dans laquelle la quantité thérapeutiquement efficace de l'au moins une isoflavone de soja est administrée au patient pédiatrique au cours d'une saison virale.

11. - Isoflavone de soja pour utilisation selon la revendication 6, dans laquelle l'au moins une isoflavone de soja est administrée quotidiennement.

12. - Isoflavone de soja pour utilisation dans une méthode de détermination de la réactivité à l'administration d'isoflavone de soja pour la prévention du développement de l'asthme chez un patient qui en a besoin, dans laquelle :
un génotype de risque de l'inhibiteur de l'activateur du plasminogène 1 (PAI-1) du patient est déterminé ; et
une quantité thérapeutiquement efficace d'au moins une isoflavone de soja est administrée au patient avant le développement de l'asthme si le génotype de risque PAI-1 est 4G4G ou 4G5G, l'au moins une isoflavone de soja étant administrée en l'absence de n'importe quels autres agents thérapeutiques ;
le génotype de risque PAI-1 4G4G ou 4G5G répondant à la prévention de l'asthme chez le patient.

13. - Isoflavone de soja pour utilisation selon la revendication 12, dans laquelle l'au moins une isoflavone de soja est administrée au patient par voie orale.

14. - Isoflavone de soja utilisée selon la revendication 12, dans laquelle l'au moins une isoflavone de soja est choisie dans le groupe consistant en la génistéine, la daidzéine et la glycitéine.
